# EUROPEAN PATENT APPLICATION

(11) **EP 4 806 561 A1**
(43) Date of publication of application: **16.09.2026**
(21) Application number: 25163794.8
(22) Date of filing: 14.03.2025
(51) Int. Cl.: B05C 17/01

(54) **DISPENSER AND APPLICATOR TIP FOR A DISPENSER**

(71) Applicant: medmix Switzerland AG, 9469 Haag (Rheintal) (CH)
(72) Inventor: DRAPER, Paul, Kenilworth, CV8 2JG (GB); McDIARMID, Ian, Leamington Spa, CV31 3JN (GB); SENIOR, James, Warwick, CV34 6QE (GB); TREWERN, Michael, Wilmcote, Stratford Upon Avon, CV37 9XN (GB); ROSEN, Hannah, Kenilworth, CV8 1QB (GB)
(74) Representative: Manitz Finsterwald Patent- und Rechtsanwaltspartnerschaft mbB

(57) **Abstract**

A dispenser extending between a front end and a rear end along a longitudinal axis, the dispenser comprising: a threaded piston rod extending and movable along the longitudinal axis; a body nut in engagement, in particular in threaded engagement, with the piston rod; a drive nut in engagement, in particular in threaded engagement, with the piston rod; and a drive tube rotatable about the longitudinal axis and at least partially accommodating the piston rod in a slidable non-engaged manner, the drive tube interacting with the drive nut, wherein a rotation of the drive tube in a first direction causes a rotation of the piston rod about the longitudinal axis by a rotation of the drive nut and the rotation of the piston rod being transferred into an axial movement of the piston rod, in particular towards the front end, by means of the engagement between the piston rod and the body nut; the dispenser further comprising a lever pivotable about a pivot axis, wherein an activation of the lever for dispense operation rotates the drive tube in the first rotation direction.

## Description

The present invention is directed at a dispenser extending between a front end and a rear end along a longitudinal axis, the dispenser comprising a threaded piston rod extending and movable along the longitudinal axis, a body nut in engagement, in particular in threaded engagement with the piston rod, a drive nut in engagement, in particular in threaded engagement, with the piston rod, and a drive tube rotatable about the longitudinal axis and at least partially accommodating the piston rod in a slidable non-engaged manner, the drive tube interacting with the drive nut, wherein a rotation of the drive tube in a first direction causes a rotation of the piston rod about the longitudinal axis by a rotation of the drive nut and the rotation of the piston rod being transferred into an axial movement of the piston rod, in particular towards the front end, by means of the engagement between the piston rod and the body nut.

Such kind of dispenser is well known in the art and is preferably employed as a multi-use dispenser to discharge a material out of a tank attached to the dispenser. Exemplary materials discharged by the dispenser from the tank storing the material are 1K materials, fluids, cremes, medical fluids, etching materials, adhesives, ointments, paints and the like. These materials may be in particular used in medical applications such as dental or surgical applications.

In such applications, it is desirable to dispense a precise amount of material. Hence, it is one object of the invention to provide a dispenser that allows for precise material dispensing.

This object is satisfied by the dispenser comprising the features of claim 1.

According to a first aspect, the invention is directed at a dispenser comprising the features of claim 1 and in particular a lever pivotable about a pivot axis, wherein an activation of the lever for dispense operation rotates the drive tube in the first rotation direction.

The invention is based on the idea that the leverage of the lever allows for applying small forces to rotate the drive tube and therefore to advance the piston rod. That is a user of the dispenser needs to apply only a rather low force to advance the piston rod. Due to the lower force used for dispensing, the lever can be precisely moved ultimately allowing for a precise material discharge.

Further advantages of the invention are defined in the dependent claims and become apparent from the following description and the accompanying drawings.

The lever can be pushed for dispense or discharge action. During such kind of activation of the lever, the lever is rotated about the pivot axis in a first direction of rotation from an initial position into an activated position in which the lever is depressed, i.e. moved radially towards the longitudinal axis of the dispenser.

Consequently, a counter rotation of the lever in a second direction opposite to the first direction moves the lever from the activated position into its initial position.

Preferably, the pivot axis of the lever is fixed with regard to the remaining parts of the dispenser. That is the pivot axis does not move with regard to the remaining parts of the dispenser, in particular as the lever is rotated. In this regard, it is noted that the pivot axis and the longitudinal axis may be aligned at least substantially perpendicular each other.

The drive tube preferably comprises an axially extending passage which accommodates the piston rod in a slidable non-engaged manner. In this regard, it is noted that the accommodation of the piston rod in the drive tube in a slidable non-engaged manner means that no engagement means are formed between the piston rod and the drive tube such that, without any further parts of the dispenser except for the piston rod and the drive tube, the piston rod can slide with regard to the drive tube. In particular if the drive tube comprises two open axial ends of the passage, the piston rod can slide through the drive tube without hindrance. Furthermore, due to the slidable non-engaged accommodation, the piston rod is also allowed to rotate relative to the drive tube.

However, it is also noted, that an outermost surface of the piston rod may bear against an inner wall of the drive tube's passage, such that the piston rod is supported by the drive tub, in particular against substantial tilting of the piston rod with respect to the longitudinal axis.

By means of definition, the front end of the dispenser may also be referred to as distal end of the dispenser, whereas the rear end of the dispenser may also be referred to the proximal end of the dispenser. In this regard, the front end of the dispenser is that part thereof which is used to dispense material, whereas the rear end refers to a portion opposite of the front end when viewed in direction of the longitudinal axis. Accordingly, a dispensing direction points from the rear end towards the front end. Furthermore, the front end of the dispenser is that end of the dispenser that in use of the dispenser is closest to the application site, which may be for example the teeth or gums of a patient.

Preferably the piston rod and the drive nut and/or the body nut are in respective threaded engagement. It is to be understood that the respective threads extend about the longitudinal axis in a spiral manner. However, in a special case of the threaded engagement, one of the engagements of the piston rod with drive nut or the body nut is in a splined engagement, with the respective splines extending at least substantially parallel to the longitudinal axis. Such kind of configuration corresponds to a threaded engagement in which the respective threads have an infinite pitch. It is understood that the respective other engagement of the piston rod and the body nut or drive nut is in conventional spiral like manner.

In the spiral like configuration, the pitch of the threaded engagement between the piston rod and the drive tube and the pitch of the threaded engagement between the piston rod and the body nut may be the same or different.

In particular, according to a preferred embodiment, the piston rod is in threaded engagement with each of the drive nut and the body nut, the respective threads of the drive nut and the body nut being counter directed and a pitch of the drive nut thread is the same as or different to a pitch of the body nut thread, in particular a drive nut thread being larger than a body nut thread. By using different pitches, the advantage of the piston rod can be adjusted.

In order to transfer a motion between the lever and the drive tube, the dispenser preferably comprises transmission means. In this regard the transmission means may allow for a bidirectional motion transfer between the lever and the drive tube. That is motion from the lever can be transferred onto the drive tube and motion from the drive tube can be transferred onto the lever.

In particular, rotation of the lever in the first direction may rotate the drive tube in a first direction. A rotation of the lever in a second direction opposite to the first direction may correspond to a rotation of the drive tube in a second direction opposite to its first direction of rotation.

The transmission means generally may comprise a geared configuration, such as for instance a worm gear.

However, in a preferred embodiment, the transmission means comprises a bevel gear associated with the lever and meshing with a corresponding bevel gear associated with the drive tube.

The lever and the bevel gear associated with the lever may be formed as a single piece, allowing for a sufficient force transfer. However, the bevel gear and the lever may also be separate pieces, with the bevel gear firmly attached to the lever, for example by using an adhesive and/or interference fit, allowing for an easy assembly.

Furthermore, the drive tube and the bevel gear associated with the drive tube may be formed as a single piece, allowing for a sufficient force transfer. However, the bevel gear and the drive tube may be separate pieces, with the bevel gear firmly attached to the drive tube, for example by using an adhesive and/or interference fit and/or force fit, enabling an easy assembly.

Preferably, the bevel gears are straight bevel gears which are easy to produce. However, the bevel gears may also be a spiral bevel gear enabling a smoother motion transmission with less vibration and less noise.

In order to equally transmit force from the lever onto the drive tube in a balanced manner, the transmission means may comprise two counter rotating bevel gears associated with the lever and two counter rotating bevel gears associated with the drive tube. The bevel gears of the drive tube may be arranged on oppositely averted side surfaces of the drive tube.

According to a preferred configuration, rotation of the drive tube in the first direction is transmitted onto the drive nut by means of a drive ratchet. In other words, the drive tube indirectly interacts with the drive nut via the drive ratchet.

In order to transmit rotation from the drive tube onto the drive nut, the drive ratchet is preferably rotationally fixed with regard to the drive tube and the drive ratchet comprises ratchet teeth interacting with corresponding ratchet teeth formed on the drive nut. In this regard, it is noted that the ratchet teeth of the drive ratchet and ratchet teeth of the drive nut are configured in a manner that rotation of the drive tube in the first direction is transmitted into a rotation of the drive nut in the same direction.

Rotational fixation of the drive ratchet with regard to the drive tube may be achieved for instance by means of a form fit connection between the drive tube and the drive ratchet, such as for example using at least one protrusion and/or at least one recess formed at the drive tube interacting with at least one corresponding recess and/or protrusion formed on the drive ratchet. In particular, the drive ratchet and the drive tube are rotationally fixed by means of a splined form fit connection, with the splines of the drive tube and the splines of the drive ratchet extending at least substantially parallel to the longitudinal axis.

Preferably the form fit connection means may be formed on an outer surface of the drive tube and the corresponding form fit connection means of the drive ratchet may be formed on an inner surface of the drive ratchet radially surrounding the outer surface of the drive tube. However, the form fit connection means of the drive ratchet may be formed on an outer surface of the drive ratchet and the corresponding form fit connection means may be formed on an inner surface of the drive tube, if the drive tube radially surrounds the drive ratchet.

According to one embodiment, the drive tube is rotated in the first rotation direction against a return force of a return spring. Preferably, the return spring is a torsional spring. The torsional spring may circumferentially surround the drive tube.

Alternatively or supplementary, the lever may be pushed against a return force of a return spring. The return spring may be a compression spring, in particular that is compressed as the lever is depressed.

Either one of the return springs or both return springs allow for bringing the drive tube and/or the lever into their respective initial position to enable further dispensing action. In this regard, as it is possible to bidirectionally transmit motion between the lever and the drive tube, only one of the return springs, i.e. the return spring directly associated with the drive tube or the return spring directly associated with the lever, is sufficient to return the lever and the drive tube into their respective initial positions.

According to one embodiment, the return force of the return spring directly associated with the drive tube is adjustable. For this purpose, the dispenser may comprise a preload means. The preload means may allow for compressing the return spring more or less by varying a position of the preload means. When a desired return force has been adjusted, the position of the preload means can be fixed, in particular with regard to the housing. Using the preload means allows for tuning a force to push the lever, since the lever indirectly interacts with the return spring directly associated with the drive tube via the transmission means.

Preferably, the body nut is rotationally fixed during dispense operation. However, the body nut may be allowed to freely rotate during resetting of the piston rod.

A rotational fixation of the body nut during dispense operation transforms the rotational movement of the piston rod in the first direction of rotation into an axial movement of the piston rod. Preferably this advances the piston rod towards the front end of the dispenser, thereby discharging material out of a tank attached to the dispenser.

It is noted that a tank attached to the dispenser is a part of the dispenser.

Free rotational movement of the body nut during resetting of the piston rod enables an easy axial movement of the piston rod towards the rear end. In this regard it is noted that resetting of the piston rod generally refers to an axial displacement of the piston rod towards the rear end of the dispenser.

Transition between a rotationally fixed body nut and a freely rotatable body nut may be achieved by means of a clutch. The dispenser therefore preferably comprises a clutch moveable, between a disengaged position and an engaged position, the body nut allowed to freely rotate in the disengaged position of the clutch and the body nut being rotationally fixed in the engaged position of the clutch.

Preferably, the clutch is axially movable with respect to the longitudinal axis between the engaged position and the disengaged position.

In order to prevent a backward movement of the drive nut as well as the piston rod, i.e. to prevent a rotation of the piston rod in the second rotation direction during lever release, the dispenser may comprise a counter ratchet blocking a rotation of the drive nut in the second direction opposite to the first direction of rotation.

Preferably, the counter ratchet comprises ratchet teeth interacting with corresponding ratchet teeth formed on the drive nut. The counter ratchet may be rotationally fixed with regard to a housing of the dispenser, by a form fit connection between the counter ratchet and the housing, such as for example using at least one protrusion and/or at least one recess formed at the counter ratchet interacting with at least one corresponding recess and/or protrusion formed at the housing. In particular, the counter ratchet and the housing are rotationally fixed by means of a splined form fit connection, with the splines of the counter ratchet and the splines of the housing extending at least substantially parallel to the longitudinal axis.

It is to be understood that the counter ratchet allows free rotational movement of the drive nut during dispense operation. However, the counter ratchet rotationally fixes the drive nut during resetting of the piston rod. The piston rod is then simply pushed through the drive nut as the piston rod is moved rearwards during resetting, in particular since the body nut is allowed to freely rotate during resetting of the piston rod.

In order to achieve blocking of the drive nut during piston rod resetting, the respective blocking directions of the drive ratchet and the counter ratchet may be effective in the same direction.

According to a further embodiment, the dispenser may comprise a spring that biases the piston rod towards the front end. This enables the piston rod to engage with a plunger accommodated in the tank attached to the dispenser. Consequently, immediate discharge action is possible as the lever is depressed.

The spring may be at least partially, preferably entirely, accommodated in the drive tube. This enables a compact set-up of the dispenser. In particular this allows for a smaller diameter of the dispenser. The spring may directly act on the piston rod or by a transmitter element arranged axially between the piston rod and the spring.

The spring acting on the piston rod may be a compression spring.

Furthermore, the spring acting on the piston rod may also be referred to as auto-prime spring.

It is to be understood that the spring force of the spring is chosen that the piston rod is not urged forwards to the front end, when a filed tank is attached to the dispenser. This ensures that material stored in the tank is not dispensed by the mere spring force of the spring, i.e. dispense operation is only achieved by activation of the lever. **In** particular, a forward urging of the drive rod by means of the spring alone can be prevented by the friction arising from the threaded engagement of the piston rod with both of the drive nut and the rotationally fixed body nut.

According to a first item of a second aspect of the invention, the invention is also directed to a further dispenser, the dispenser comprises a housing extending between a front end and a rear end along a longitudinal axis, the housing comprising a side wall extending along the longitudinal axis; the dispenser further comprising a piston rod axially movable relative to the housing along the longitudinal axis; and the dispenser further comprising an indicator means interacting with the piston rod, the indicator means indicating an axial position of the piston rod relative to the housing.

In this connection it is mentioned that the dispenser of the second aspect may comprise at least some of the features as described with regard to the dispenser of the first aspect. Likewise, the dispenser of the first aspect may comprise at least some of the features described with regard to the dispenser of the second aspect. In particular, any of the items of the dispenser of the second aspect described hereinafter may be combined with any of the features described above with the regard to the dispenser of the first aspect. Furthermore, any of the features of the first dispenser may be combined with any of the items of the dispenser according to the second aspect.

Using an indicator means in a dispenser as described herein allows for a precise dispensing of a material, as the travel of the piston rod can be seen by the user. In particular, as the axial position of the piston rod correlates with the material discharged from the storage device, such as the tank, the travel of the piston rod can be used to visualize how much material has already been discharged from the storage device. In order to enhance the visualization, the dispenser comprises an indicator means interacting with the piston rod. The indicator means thus indicates an axial position of the piston rod and therefore also how much material has been dispensed from the storage device.

According to a second item in accordance with the first item of the second aspect, the indicator means may move together with piston rod in direction of the longitudinal axis. This gives a direct feedback on the travel distance of the piston rod by the indicator means.

According to a third item in accordance with the first or second items of the second aspect, the piston rod may be rotatable relative to the indicator means and/or the housing. This allows for only the piston rod to be rotated and results in less frictional forces during rotation of the piston rod compared to a scenario in which both of the piston rod and the indicator means are rotated together.

It is noted that the piston rod can be rotated in a manner as described beforehand, in particular using a rotation mechanism, with the rotation of the piston rod being transferred into an axial movement thereof by using the body nut.

Furthermore, in order to allow the piston rod to be rotatable relative to the indicator means and/or the housing, the indicator means and the piston rod and/or the housing may be formed as different parts. This allows for an easy production of these parts and an easy assembly thereof.

According to a fourth item in accordance with any of the other items of the second aspect, the indicator means is preferably rotationally fixed with regard to the housing and/or the piston rod. This allows for the indicator means to move axially together with the piston rod as the piston rod is axially moved, without rotating the indicator means during rotation of the piston rod. The indicator means is thus not rotated out of sight and remains visible for a dispenser user.

According to a fifth item in accordance with any of the other items of the second aspect, the indicator means may comprise at least one guidance means axially guided by at least one complementary guidance means formed at the housing, in particular the complementary guidance means of the housing extending in direction of the longitudinal axis. Such a configuration of the guidance means safely guides the indicator means during axial movement.

According to a sixth item in accordance with the fifth item of the second aspect, the guidance means of the indicator means may be formed at an outer circumference of the indicator means, and/or the guidance means of the housing may be formed on an inner side of the side wall. Such kind of guidance means is easy to produce.

According to a seventh item in accordance with the fifth or sixth items of the second aspect, the guidance means of the indicator means may be a protrusion and the guidance means of the housing may be a groove extending in direction of the longitudinal axis. Additionally or supplementary, the guidance means of the indicator means may be a notch and the guidance means of the housing may be a rib extending in direction of the longitudinal axis. It should be understood that the groove and/or the rib has an axial length that basically corresponds to the axial travel of the piston rod.

According to an eighth item in accordance with any of the other items of the second aspect, the housing may comprise a window, in particular the window forming a cut-out portion at the side wall of the housing. This enables visualization of the indicator means, in particular if the indicator means is accommodated in the housing. Preferably the window has at least about the same axial length as an axial travel of the indicator means interacting with the piston rod. However, the axial length of the window may be shorter than the axial travel of the indicator means, as long as the indicator means at least in sections remains visible through the window. The window may also be segmented in axial direction such that the indicator means can be only seen in sections, as the indicator means is axially moved.

The window may be formed as an open cut out portion of the housing. However, for hygienic reasons the window is preferably closed by a transparent material.

It is also noted that the housing of the dispenser may be formed at least in sections of a transparent material. In particular, that part of the housing accommodating the indicator means may be formed of a transparent material.

According to a ninth item in accordance with the fifth to eighth items of the second aspect, the window may form a part of the guidance means present at the housing. In other words, the window not only allows for visualization of the indicator means, but also enables guidance of the indicator means. In particular, the window may comprise said groove or rib. Furthermore, the guidance means of the window may transition into a guidance means formed at the remaining part of the housing.

According to a tenth item in accordance with any of the other items of the second aspect, the dispenser may comprise a spring biasing the piston rod towards the front end of the housing. This can be the auto-prime spring mentioned above. Furthermore, as detailed below, the spring may act on the indicator means, thereby biasing the indicator means against the piston rod. This ensures that the indicator means travels together with the piston rod in axial direction.

According to an eleventh item in accordance with the tenth item of the second aspect, one side of the spring may bear against the rear end of the housing and the other side of the spring may bear against the indicator means, in particular a radially outwardly protruding collar formed at the indicator means. This ensures that the indicator means is in contact with the piston rod. In particular, this configuration ensures that the indicator means axially travels with the piston rod towards the front end of the dispenser. It is understood that the piston rod and the indicator means are moved against the spring force of the spring as the piston rod is axially moved towards the rear end of the dispenser, i.e. during resetting of the piston rod.

According to a twelfth item in accordance with the eleventh item of the second aspect, the indicator means may comprise a cover at least partially surrounding the spring in circumferential direction, in particular the cover also at least in sections covering the spring in direction of the longitudinal axis. The cover enhances visibility of the indicator means and may at least in sections hide the spring.

According to a thirteenth item in accordance with the fifth to twelfth items of the second aspect, the cover may form the guidance means of the indicator means. The cover has therefore a beneficial double function. In particular, the cover may be the protrusion formed on the indicator means guided in the groove formed at the housing and/or the window.

According to a fourteenth item in accordance with one of the eighth to thirteenth items of the second aspect, only the piston rod may be visible through the window, when the piston rod is in its fully retracted rear end position. That is, the indicator means is hidden by the housing, when the piston rod is in its fully retracted rear end position. This indicates the user that the storage device, such as the tank, is completely full.

Additionally or supplementary, only the spring may be visible through the window, when the piston rod is in its fully extended front end position. That is, the indicator means is hidden by the housing, when the piston rod is in its fully extended front end position. This indicates the user that the storage device is completely emptied.

Additionally or supplementary, the indicator means may be visible through the window, when the piston rod is in a position between its fully retracted rear end position and its fully extended front end position.

According to a fifteenth item in accordance with any of the other items of the second aspect, at least one of the piston rod and indicator means may be coloured, in particular the piston rod and the indicator means being coloured in the same colour or in different colours. Colouring of the piston rod and/or the indicator means enhances visibility thereof.

According to a first item of a third aspect of the invention, the invention also concerns an applicator tip, in particular for a dispenser as described in the foregoing, the applicator tip extending between a rear end and a front end along an axial direction, the rear end being open and the front end comprising a closed front wall transitioning into a side wall circumferentially extending about the axial direction, the side wall being radially deflectable with regard to the axial direction and the side wall comprising a connection member formed on an inner side of the side wall.

At this point it is mentioned that the applicator tip as described with regard to the third aspect may be used in combination with a dispenser of the first aspect and/or the dispenser of the second aspect. **In** particular, the applicator tip may be connected to a further component of the dispenser, such as for instance a housing of the dispenser and/or a shielding accommodating the housing of the dispenser and/or a material storing tank received in the dispenser. **In** particular, any of the items of the third aspect may be combined with any of the features described with the regard to the first aspect and/or the items described with regard to the second aspect of the invention.

It is noted that the axial direction of the applicator tip and the longitudinal axis of the dispenser are aligned at least substantially parallel, preferably coaxially, to each other, when the applicator tip is connected to the further component of the dispenser.

According to a second item in accordance with the first item of the third aspect, the side wall may be spring elastically radially deflectable. This lets the applicator tip to snap back into its initial shape after being deflected for the purpose of attaching to or detaching from the further component of the dispenser.

According to a third item in accordance with the first item or second item of the third aspect, the side wall may be formed of an elastic material, in particular a spring elastic material, and/or wherein thickness of the side wall may be chosen to allow for an elastic deflection, in particular a spring elastic deflection, of the side wall. Such a configuration allows for the side wall to be radially deflectable, in particular spring elastically deflectable.

According to a fourth item in accordance with any of the other items of the third aspect, the connection member may be a part of a connection assembly selected from one of a snap fit type, a bayonet type or a screw type connection assembly. A bayonet type or a screw type connection assembly would also allow to form the side wall of a more rigid material.

According to a fifth item in accordance with any of the other items of the third aspect, the connection member may comprise a protrusion protruding radially inwardly from the inner side of the side wall, in particular the protrusion at least in sections, preferably entirely, extending in circumferential direction of the side wall. The protrusion is configured to be received in a corresponding recess of the further component of the dispenser.

Alternatively or supplementary, the connection member may comprise a recess formed on the inner side of the side wall, in particular the recess at least in sections, preferably entirely, extending in circumferential direction of the side wall. The recess is configured to receive a corresponding protrusion of the further component of the dispenser.

If the protrusion and/or the recess extends entirely in the circumferential direction, then it is possible to fully rotate the applicator tip about the axial direction. That is the applicator tip can be rotated about 360°.

Generally, the applicator tip may comprise a passage extending through the front wall. Material can be discharged through the passage.

In particular, according to a sixth item in accordance with any of the other items of the third aspect, the applicator tip may comprise a cannula. That is, the cannula forms the passage extending through the front wall.

The applicator tip and the cannula can be formed as single piece or may be formed as separate pieces.

The cannula may be straight or bend or bendable.

The applicator tip may also comprise a mixer or means for connecting a mixer to the applicator tip. This is beneficial if the dispenser is used to discharge more than one material, in particular at least two materials that upon contact with each other react.

However, it is also possible that the applicator tip does not comprise any passage. The applicator tip can then be used as a closure cap.

According to a seventh item in accordance with any of the other items of the third aspect, the side wall may be arranged radially inwardly with respect to an outer shell wall, the shell wall at least in sections, preferably entirely, circumferentially surrounding the side wall. The shell wall thus protects the side wall.

According to an eighth item in accordance with the seventh item of the third aspect, the side wall may be more flexible or formed of a more flexible material than the shell wall. The shell wall may then be used as a shielding, whereas the side wall allows for a connection to the further component of the dispenser.

According to a ninth item in accordance with the seventh or eighth item of the third aspect, a radial gap may be formed between the shell wall and the side wall. This allows for radial deflection of the side wall relative to the shell wall.

Additionally or supplementary, the side wall and the shell wall respectively may emanate from the front wall; or the shell wall may emanate from the side wall.

According to a tenth item in accordance with the other items of the third aspect, a plug may extend axially, in particular coaxially, from an inner side of the front wall towards the rear end of the applicator tip. In this regard it is noted that the passage or cannula may not only extend through the front wall, but also through the plug.

According to another embodiment, the plug may be closed, for example when the applicator tip is used as a closure cap.

According to an eleventh item of the third aspect, the invention also refers to a system comprising an applicator tip in accordance with any one of the other items of the third aspect and a further component of a dispenser, such as a housing of the dispenser and/or a shielding accommodating the housing of the dispenser and/or a material storing tank received in the dispenser, the further component comprising an outlet opening and the further component of the storage system comprising a connection member of the connection assembly, the connection member of the further component is a counter connection member formed at least in sections complementary to the connection member of the applicator tip.

It is noted that the dispenser can be at least one of the dispensers of the first or second aspects.

Furthermore, a longitudinal axis of the further component and the axial direction of the applicator tip may be aligned at least substantially parallel, in particular coaxial, to each other when connected.

According to a twelfth item in accordance with the eleventh item of the third aspect, the applicator tip may be connected to the further component of the dispenser by a predetermined breaking means allowing for a detachment of the applicator tip from the further component of the dispenser upon application of a predetermined breaking force. This allows for a user convenient provision of the further component and the applicator tip. That is, the user can use the applicator tip appropriate for the further component after breaking it away from the further component. This further ensures operational safety as the correct applicator tip is used in combination with the corresponding further component of the dispenser.

According to a thirteenth item in accordance with the twelfth item of the third aspect, the predetermined breaking means may be formed between an axial end section of the further component of the dispenser, in particular comprising the outlet opening, and the applicator tip, in particular the front wall of the applicator tip, preferably an outer side of the front wall of the applicator tip or an inner side of the front wall of the applicator tip.

If the predetermined breaking means is formed between the inner side of the front wall of the applicator tip and the axial end section of the further component, the applicator tip can be broken away from the further component for example by twisting the applicator tip about the axial direction. Further twisting of the applicator tip can then bring the applicator tip in a position for further engagement with the further component, such that the applicator tip is ready to use.

If the predetermined breaking means is formed between an outer side of the front wall of the applicator tip and the axial end section of the further component, then the applicator tip needs to be flipped over and attached to the further component such that the further component is at least partially received in a cavity formed by the front wall and the side wall, thereby rendering the applicator tip ready to use.

According to a fourteenth item in accordance with the twelfth or thirteenth item of the third aspect, the predetermined breaking means may form a seal closing the outlet opening of the further component of the dispenser, when the applicator tip is connected to the further component of the dispenser by the predetermined breaking means. This may prevent material from escaping from the further component and/or other contamination to enter into the further component, when the applicator tip is attached to the further component by means of the predetermined breaking means.

According to a fifteenth item in accordance with the tenth item in combination with any of the twelfth to fourteenth items of the third aspect, the plug may extend into the outlet opening of the further component, in particular such that an outer surface of the plug tightly, in particular gas and/or fluid tightly, bears against an inner surface of the outlet opening. This configuration prevents any leakage between the further component and the applicator tip and allows for the material to only discharge through the passage or the cannula if the applicator tip is provided therewith. If the applicator tip forms a closure cap, then the configuration described in the fifteenth item enhances closing and sealing behavior of the applicator tip.

In the following, exemplary embodiments and functions of the present disclosure are described herein in conjunction with the drawings, showing schematically:
- Fig. 1: a perspective explosion view of a multi-use dispenser comprising a shielding;
- Fig. 2: a perspective explosion detail of the multi-use dispenser of Fig. 1;
- Fig. 3: a cross-sectional side view of an assembled multi-use dispenser without an applicator tip;
- Fig. 4: a cross-sectional view of the assembled multi-use dispenser without shielding;
- Fig. 5: a perspective detail of the multi-use dispenser;
- Fig. 6: a perspective partial cross-sectional detail of the multi-use dispenser;
- Fig. 7: a cross-sectional detail of the multi-use dispenser, with a clutch in a disengaged state;
- Fig. 8: the cross-sectional detail of Fig. 7, with the clutch in an engaged state;
- Fig. 9a: a perspective detail of a tank storable in the multi-use dispenser;
- Fig. 9b: a perspective detail of the clutch of Figs. 7 and 8;
- Fig. 10: a perspective cross-sectional showing an interaction of the parts shown in Figs. 9a and 9b;
- Fig. 11: a semi transparent view of yet another detail of the multi-use dispenser;
- Fig. 12: a perspective view of an applicator tip of the multi-use dispenser;
- Fig. 13: a perspective view of an outlet section of a tank engageable with the applicator tip of Fig. 12;
- Fig. 14: a longitudinal sectional side view of a multi-use dispenser according to a further embodiment;
- Fig. 15: a partial semi transparent perspective view of parts of the dispenser of Fig. 14;
- Fig. 16: a perspective view of a detail of the some of the parts of Fig. 15;
- Fig. 17: a longitudinal sectional side view of a first configuration of an applicator tip and a tank for use with a multi-use dispenser;
- Fig. 18: the applicator tip and the tank of Fig. 17 in a second configuration;
- Fig. 19: a longitudinal sectional view of a further embodiment of an applicator tip in combination with a tank;
- Fig. 20: a longitudinal sectional view of yet a further embodiment of an applicator tip.
- Fig. 21: a longitudinal sectional view of even a further embodiment of an applicator tip,
- Fig. 22: a side view of a rear part of a dispenser comprising an indicator means; and
- Fig. 23: a perspective view showing a detail of the indicator means of Fig. 22 interacting with a piston rod and a spring.

In the following, a multi-use dispenser 10 and advantageous aspects thereof are described with reference to the accompanying drawings. Statements made with regard to direction and use are made with reference to the drawing.

The multi-use dispenser 10 may also be referred to as dispensing device, dispensing system or shortly dispenser 10 and is typically applicable to dispense a material used in medical and dental applications. In particular, the material dispensable by the multi-use dispenser 10 may be for example at least one of 1K materials, fluids, cremes, medical fluids, etching materials, adhesives, ointments, paints and the like.

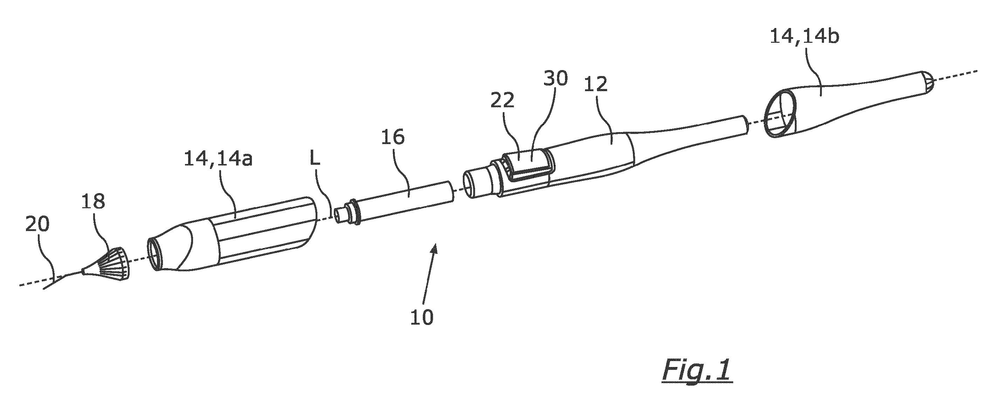

Figs. 1 to 13 show a first embodiment of a dispenser 10 and advantageous details thereof.

As can be seen from Fig. 1, the dispenser 10 comprises a housing 12. The housing 12 may be formed as a single piece or may comprise multiple parts.

The housing 12 is at least partly receivable inside a shielding 14 used as a protection against external influences, such as fluids, dust, dirt or the like. The shielding 14 as shown in Fig. 1 comprises a front part 14a and a rear part 14b connectable to the front part 14a. The front part 14a and the rear part 14b are connectable, for example by means of a snap-lock connection or a threaded joint. However, the shielding 14 may also be formed as a single piece. Furthermore, the shielding 14 may be formed by at least two parts connected or connectable parallel to the along the longitudinal direction.

Furthermore, the housing 12 is configured to at least partly receive a tank 16 therein.

The housing 12, the shielding 14 and the tank 16 each extend into a longitudinal direction defining a longitudinal axis L.

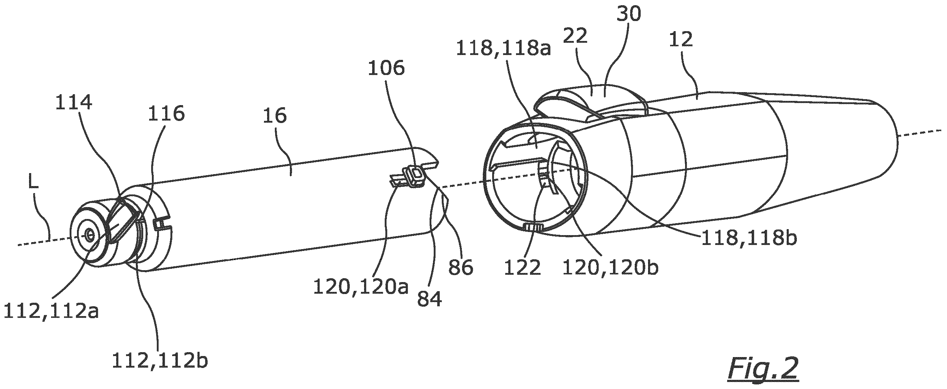

The tank 16 is connectable to the housing 12 by a bayonet type connection mechanism (Fig. 2). For this purpose the tank 16 comprises at its outer circumferential surface at least one protrusion 106 that, upon assembly, is guided in a L-shaped groove 118 formed on an inner wall of the housing 12. The L-shaped groove comprises an axial portion 118a extending in direction of the longitudinal axis L and a circumferential portion 118b extending in a circumferential direction of the inner wall of the housing 12.

In the present embodiment, the tank 16 comprises two protrusions 106 formed in a diametrically opposed manner on the outer circumferential surface of the tank 16. In this context, the housing 12 comprises two corresponding L-shaped grooves 118 present in an inner wall of the housing 12.

In order to safely retain the tank 16 inside the housing 12, when assembled, the tank 16 and the housing 12 comprise retaining means 120.

A first retaining means 120a is formed as a radially outwardly projecting protrusion at the outer circumferential surface of the tank 16, whereas a second retaining means 120b is formed as a radially inwardly projecting protrusion at the inner circumferential surface of the housing 12. Furthermore, in an entirely assembled state, the first retaining means 120a of the tank 16 rests in a pocket 122 formed on the inner circumferential surface of the housing 12.

As can be seen from Fig. 2, the first retaining means 120a is arranged axially adjacent to the protrusion 106 as seen in the longitudinal axis L. Correspondingly, the second retaining means 120b and the pocket 122 are arranged axially adjacent to the circumferential portion 118b of the L-shaped groove 118.

In order to connect the tank 16 to the housing 12, the tank 16 is axially inserted into the housing 12 such that the protrusion 106 is guided in the axial portion 118a of the groove 118. When the tank 16 is fully axially inserted, the tank 16 is then rotationally twisted, such that the protrusion 106 is guided in the circumferential portion 118b of the groove 118 (in Fig. 2 counter-clock wise). At the same time, the first retaining means 120a of the tank 16 passes by the second retaining means 120b of the housing 12 and ultimately comes at rest in the pocket 122, when the tank 16 is entirely connected to the housing 12. In this state, the retaining means 120a, 120b hinder a backwards rotation of the tank 16, thereby safely connecting the tank 16 to the housing 12. It is to be understood, that the tank 16 can be removed from the housing 12 by being moved in a manner opposite to the above described assembly procedure. It is also noted that hand strength of the user is sufficient to pass the first retaining means 120a by the second retaining means 120b.

The tank 16 is configured to store a material, such as the ones discussed above.

It is noted that, generally speaking, a front part refers to a portion of the dispenser 10 from which the material can be discharged, whereas a rear part refers to a portion opposite of the front part when viewed in direction of the longitudinal axis L. Accordingly, a dispensing direction points from the rear part towards the front part.

Furthermore, a front part of the housing 12 of the dispenser 10 may also refer to that part of the housing 10 into which the tank 16 is inserted to receive the tank 16 at least partially within the housing 10.

In order to apply the material dispensed by the dispenser 10, for example to a tooth of a patient, the dispenser 10 comprises an applicator tip 18.

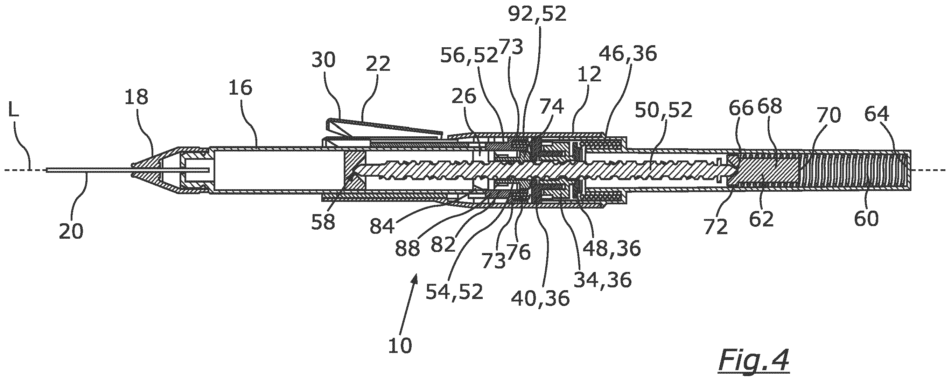

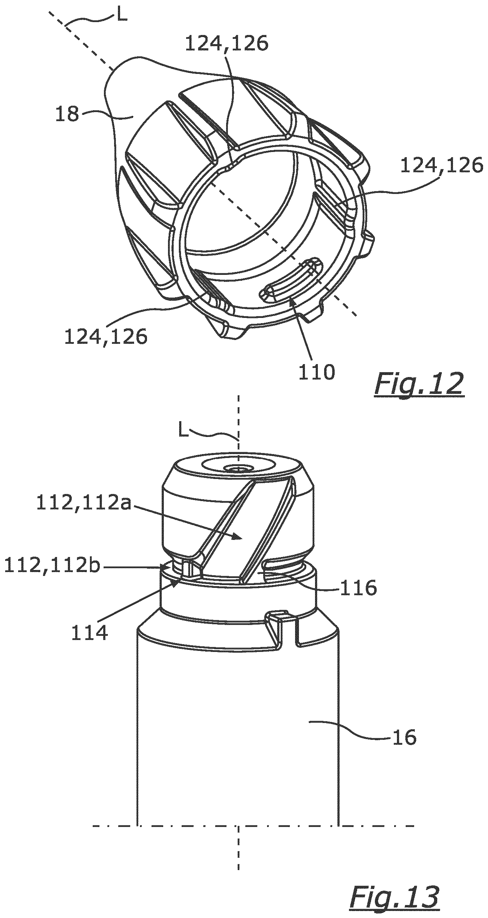

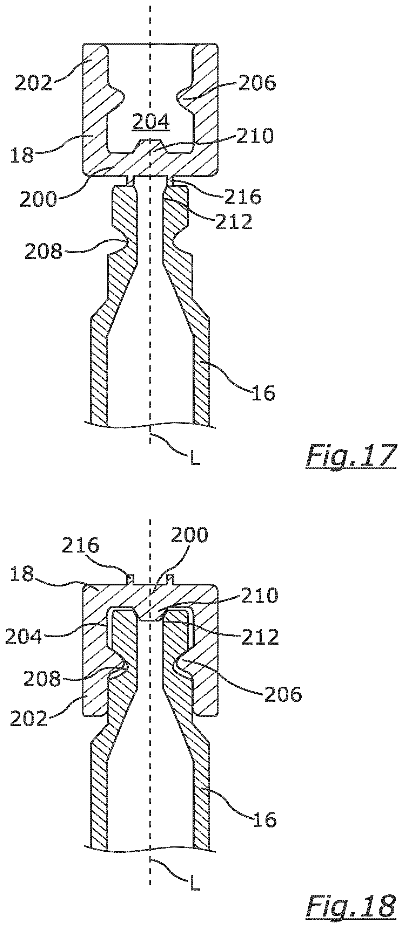

The applicator tip 18 may be connectable to either one of the housing 12, the shielding 14 or the tank 16. In this context, in Fig. 1 the applicator tip 18 is connectable to the front part 14a of the shielding 14, whereas Fig. 4 shows an embodiment in which the applicator tip 18 is connected to the tank 16. An advantageous way to connect the applicator tip 18 will be described later with regard to Figs. 12 and 13. Further aspects of an applicator tip will also be described with regard to Figs. 17 to 21.

The applicator tip 18 serves as a dispending outlet of the dispenser 10. However, the applicator tip 18 may be also configured as closure cap, such that when attached to the housing 12, the shielding 14 and in particular to the tank 16 material is retained.

The applicator tip 18 may further comprises a cannula 20. The cannula 20 allows for a more precise application of the material dispensed by the dispenser 10.

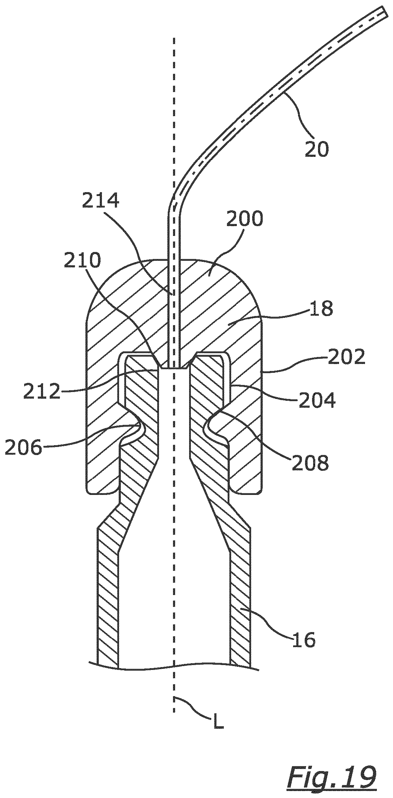

The cannula 20 may be already bent (Fig. 19) or individually bendable as indicated in Fig. 1 or may be straight as shown in Fig. 4. It is to be noted that the applicator tip 18 may be also formed without a cannula if the material is to be dispensed over larger areas than within a tooth canal or the like.

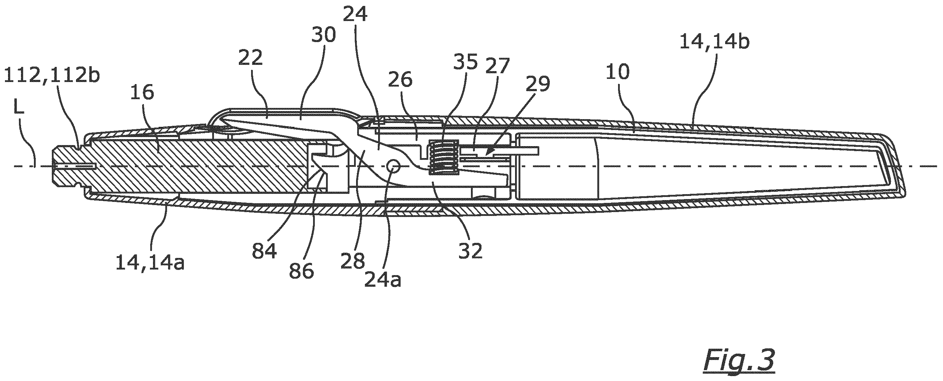

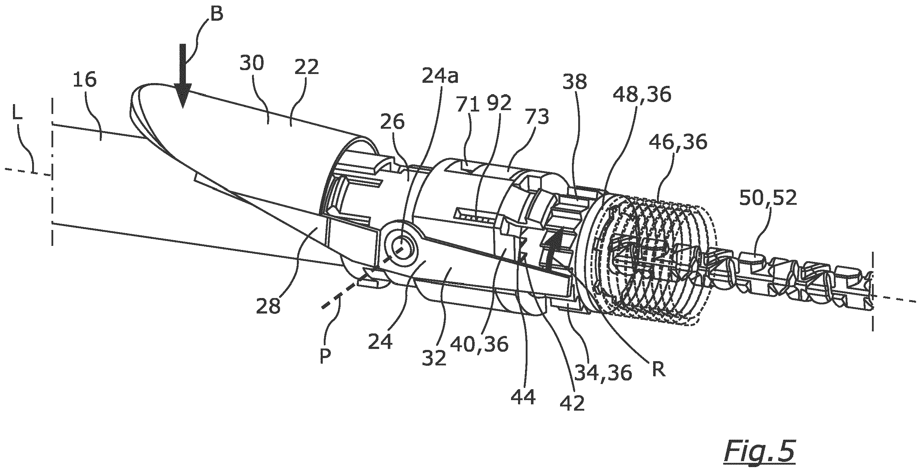

Now turning to Figs. 3 and 5 further details of the dispenser 10 will be described with reference to the other drawings.

The dispenser 10 comprises a dispense activator 22 that is movable relative to the housing 12. Activation of the dispense activator 22 allows for dispensing a certain pre-defined amount of material from the dispenser 10.

As can be best seen from Fig. 5, the dispense activator 22 comprises a two-arm lever 24. The two-arm lever 24 extends in a direction parallel to the longitudinal axis L and is pivotably supported by a fulcrum 24a to make the two-arm lever 24 pivotable about a pivot axis P. The pivot axis P is oriented perpendicular to the longitudinal axis L.

The fulcrum 24a may be formed at the inner wall of the housing 12 or, as shown in Fig. 5, on a support structure 26. The support structure 26 is receivable in the housing 12.

One arm 28 of the two-arm lever 24 is attached to a push button 30, whereas the other arm 32 of the two-arm lever 24 is configured to drive a drive nut 34 of a rotation mechanism 36. The arm 28 of the two-arm lever 24 attached to the push button 30 may also be referred to as a push arm 28 and the other arm 32 of the two-arm lever 24 engageable with the drive nut 34 may be referred to as a drive arm 32.

In order to limit a travel of the two-arm lever 24, the dispenser 10 comprises a stopping element 27 that interacts with the drive arm 32 of the two-arm lever 24 and stops a travel of the two-arm lever 24, when the drive arm 32 comes at rest at the stopping element 27. The stopping element 27 ensures that the push arm 28 of the two-arm lever 24 does not collide with the tank 16 received in the housing 12, when the push button 30 is pushed radially inwardly. Generally speaking, the stopping element 27 limits a travel of the dispense activator 22 and therefore hinders a collision of the dispense activator 22 with the tank 16 received in the housing 12.

A snap dome 29 is attached to the stopping element 27 and provides an acoustical feedback, when the drive arm 32 of the two-arm lever 24 reaches the stopping element 27. The user then knows that the dispense activator 22 has been fully activated, such that the user can release the dispense activator 22 and activate the dispense activator 22 by pushing of the push button 30 of the dispense activator 22 again for further material discharge.

Furthermore, in order to ensure a secure and proper functioning of the push button 30, the push button 30 is supported on both longitudinal sides with an arm of a lever. In detail, the push button 30 is supported on one side by means of the push arm 28 of the two-arm lever 24 and on the opposite side by an arm of a single-arm lever (not shown). The two-arm lever 24 and the single-arm lever are pivotable about the same pivot axis P.

Furthermore, the two-arm lever 24 and the single-arm lever are arranged on opposite sides of the support structure 26. However, the two-arm lever 24 and the single-arm lever may also be arranged on opposite inner wall sections of the housing 12.

As indicated above, the push arm 28 of the two-arm lever 24 interacts with the drive nut 34 of the rotation mechanism 36. For this purpose, the two-arm lever 24 comprises a cam (not shown) projection radially inwardly towards the drive nut 34 and interacting with ratchet teeth 38 formed on an outer circumferential surface of the drive nut 34.

Rotational movement of the drive nut 34 is achieved, when the push button 30 of the dispense activator 22 is pushed radially inwardly with respect to the longitudinal axis L (arrow B in Fig. 5), which results in a counter directed movement of the drive arm 32. During this movement of the drive arm 32 its cam engages with one of the ratchet teeth 38 of the drive nut 34, thereby rotating the drive nut 34 about the longitudinal axis L (arrow R in Fig. 5).

A spring 35 (Fig. 3) pushes the dispense activator 22 back into its initial position, such that the dispense activator 22 can be operated again to further rotate the drive nut 34.

In order to avoid an unwanted backwards rotational movement of the drive nut 34, the rotation mechanism 36 comprises a blocking element 40 interacting with the drive nut 34. The blocking element 40 is rotationally fixed and acts as a counter part for the drive nut 34. The blocking element 40 and the drive nut 34 together form a ratchet.

In particular, the drive nut 34 comprises further ratchet teeth 42 formed on an end face of the drive nut 34 interacting with corresponding ratchet teeth 44 formed on an end face of the blocking element 40.

As becomes apparent from Fig. 5, the blocking element 40 is connected to the support structure 26. However, the blocking element 40 and the support structure 26 may also be a single piece. That is, the blocking element 40 may be formed at the support structure 26.

During rotation of the drive nut 34, its ratchet teeth 42 pass by the ratchet teeth 44 of the blocking element 40. After rotational movement of the drive nut 34, engagement between the ratchet teeth 42 of the drive nut 34 and the ratchet teeth 44 of the blocking element 40 hinder a reverse rotational movement.

In order to ensure a safe engagement of the ratchet teeth 42, 44 of the drive nut 34 and the blocking element 40, the rotation mechanism 36 comprises a spring 46 that axially biases the drive nut 34 towards the blocking element 40 with regard to the longitudinal axis L. The spring 46 is compressed as the ratchet teeth 42 of the drive nut 34 pass by the ratchet teeth 44 of the blocking element 40 during rotation of the drive nut 34.

One end of the spring 46 bears against the housing 12, whereas the other end of the spring 46 bears against a washer 48 arranged between the spring 46 and the drive nut 34. The washer 46 ensures a safe rotational movement of the drive nut 34 during operation.

Furthermore, the drive nut 34 comprises an inner thread in threaded engagement with a threaded piston rod 50 of a dispensing mechanism 52. The threaded engagement between the drive nut 34 and the piston rod 50 allows for a transmission of the rotational movement of the drive nut 34 to a rotational movement of the piston rod 50.

The piston rod 50 further engages with a body nut 54 of the dispensing mechanism 52. For example, the piston rod 50 and the body nut 54 may be in threaded engagement. In this context, it is to be noted that the piston 50 may be double threaded, wherein one thread interacts with the drive nut 34 and the other thread interacts with the body nut 54. Preferably, the respective threads are configured in a counter running manner.

As will be described later, the body nut 54 is rotationally fixable by means of a clutch 56. In a rotationally fixed state, the body nut 54 enables a transformation of a rotational movement into a translational movement of the piston rod 50 in which the piston rod 50 moves in direction of the longitudinal axis L towards the front end of the dispenser 10.

In particular, a translational movement of the piston rod 50 brings the front end of the piston rod 50 into contact with a plunger 58 (Fig. 4) stored inside of the tank 16 when received in the housing 12. In particular, when the piston rod 50 contacts the plunger 58, activation of the dispense activator 22 results in a further translational movement of the piston rod 50, thereby pushing the plunger 58 towards a front end of the tank 16 ultimately resulting in a dispense operation. The plunger 58 is also referred to as piston 58.

In this regard, it is desirable that dispensing from the tank 16 is directly possible or at least after only a few operational clicks of the dispense activator 22 even if the tank 16 is installed or reinstalled that was partly emptied or originally only partly filled.

To achieve this, the dispenser 10 comprises a spring 60 that acts on the piston rod 50, with the spring force of the spring 60 being selected such that the piston rod 50 is urged in the dispensing direction towards the plunger 58 of the tank 16, thereby making the dispenser 10 directly ready to use after insertion of the tank 16.

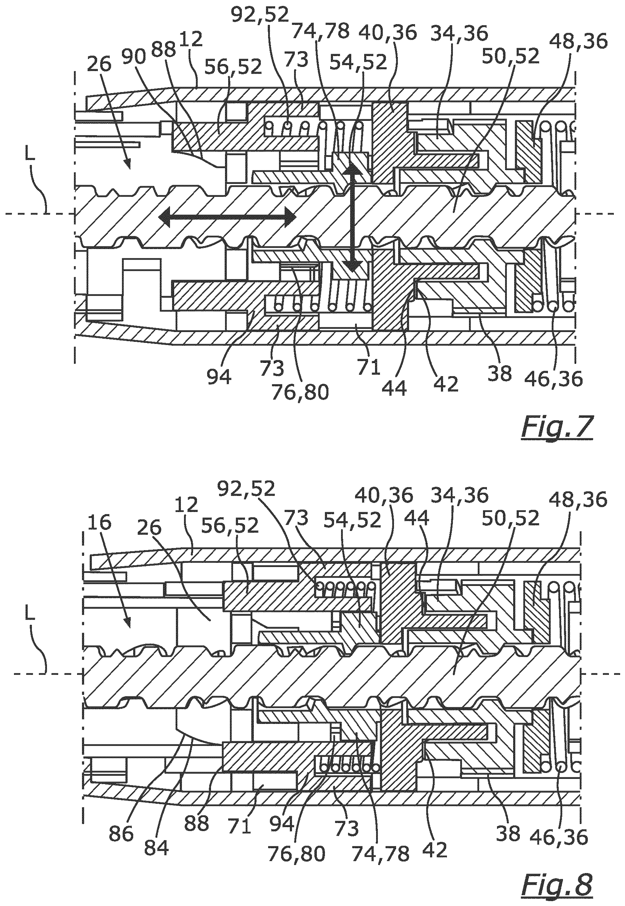

In particular, the spring 60 acts with one end on the piston rod 50 by means of a transmitter element 62, whereas the other end of the spring 60 bears against the housing 12, in particular an inner end surface 64 of the housing 12 (see Figs. 4 and 7).

The transmitter element 62 comprises a cavity 66 configured to receive a reverse end of the piston rod 50, i.e. the end of the piston rod 50 that does not engage with the plunger 58. The cavity 66 has a conical shape that enhances the coupling between the piston rod 50 and the transmitter element 62.

Furthermore, the transmitter element 62 comprises a guidance structure 68. The guidance structure 68 is set back radially inwardly with respect to a collar 72 against which the spring 60 bears.

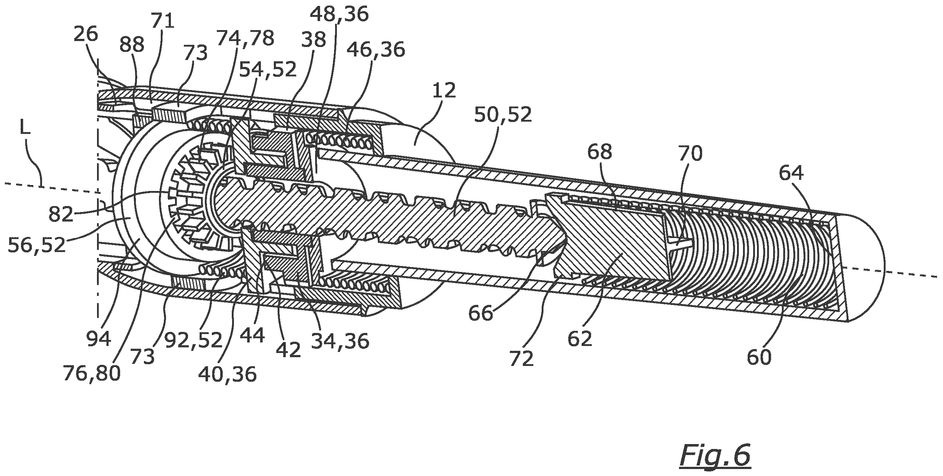

As can be seen from Figs. 4 and 6, the guidance structure 68 is surrounded by the spring 60 and prevents an unwanted tilting of the transmitter element 62, when the spring 60 is compressed or decompressed.

A rear end 70 of the guidance structure also serves as a stopper upon contact with the inner end surface 64 of the housing 12, thereby limiting a travel of the piston rod 50 towards the rear direction.

In the embodiment shown in Fig. 7, the guidance structure 68 has a cross-shaped cross-section. However, the guidance structure 68 may also have other cross-sectional shapes, for example a round cross section.

To allow the spring 60 to axially displace the piston rod 50, the dispenser 10 comprises said clutch 56 interacting with the body nut 54 such that upon disengagement of the clutch 56 the body nut 54 is allowed to freely rotate thereby enabling an easy axial displacement of the piston rod 50 by means of the spring force of the spring 60. In other words, the spring 60 pushes the piston rod 50 through the drive nut 34.

However, when the clutch 56 is engaged, the body nut 54 is rotationally fixed. The piston rod 50 is then no longer axially displaceable by the spring 60. In fact, the engagement of the piston rod 50 with both of the rotationally fixed body nut 54 and the drive nut 34 prevents an axial displacement of the piston rod 50 by means of only the spring 60. Therefore, an unwanted discharge operation caused by only the spring 60 is avoided. Nevertheless, the piston rod 50 can still be axially displaced by means of the rotation mechanism 36 activated by the dispense activator 22.

It is noted that the clutch 56 is rotationally fixed. However, to still allow a dis- or engagement with the body nut 54, the clutch 56 is axially displaceable with regard to the longitudinal axis L.

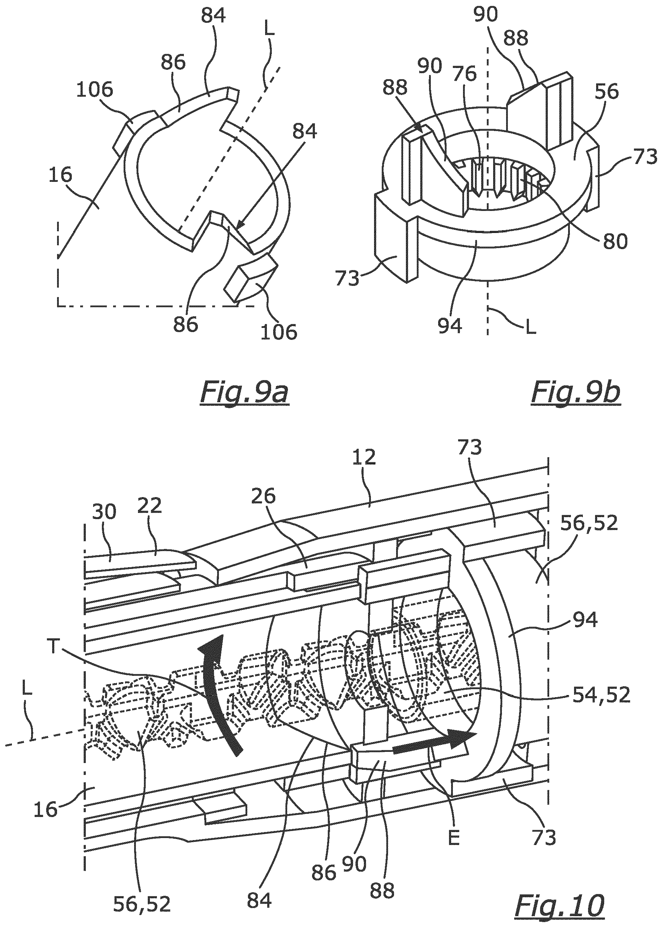

For a rotational fixation as well as a guidance during axial displacement, the clutch 56 comprises guidance means 73 formed at its outer circumferential surface (see in particular Fig. 9b). The guidance means 73 are respectively guided in a longitudinally extending grooves 71 present in the support structure 26 (Fig. 5). The groove may be also formed on an inner surface of the housing 12.

In the present embodiments, the clutch 56 comprises two guidance means 73 arranged at the outer circumferential surface of the clutch 56 in a diametrically opposed manner. The clutch 56 may also comprise only one guidance means 73 or more than two guidance means 73, like three, four or more guidance means 73.

An engagement between the body nut 54 and the clutch 56 is achieved by engagement means 74 formed at the body nut 54 and corresponding engagement means 76 formed at the clutch 56.

In the embodiment shown in Fig. 7, the engagement means 74 of the body nut 54 are formed as teeth 78 extending radially outwardly from a circumferential surface of the body nut 54.

In a corresponding manner, the engagement means 76 of the clutch 56 are formed as teeth 80 extending radially inwardly into a passage 82 extending through the clutch 56 and configured to receive the body nut 54. In particular, the passage 82 of the clutch 56 is dimensioned in such a manner that in an engaged state, the body nut 54 is at least partly received therein.

Furthermore, it is to be understood, that in the engaged state, the teeth 78 of the body nut 54 mesh with the teeth 80 of the clutch 56, thereby hindering rotational movement of the body nut 54.

It is noted, that an engagement between the clutch 56 and the body nut 54 is also possible with one tooth extending into the passage 82 of the clutch interacting with one cavity formed at the body nut 54. In a similar manner, the body nut 54 may comprise a radially outwardly extending tooth interacting with a cavity formed in the passage 82 of the clutch 56.

The body nut 54 and the clutch 56 can also be configured in the opposite manner, i.e. the body nut 54 may comprise a passage configured to at least partially receive the clutch 56 therein, when the clutch 56 is engaged. In this configuration, at least one tooth and/or at least one cavity is formed in the passage of the body nut 54 interacting with at least one corresponding cavity and/or tooth formed at the clutch 56, when the clutch 56 and the body nut 54 are engaged.

It is further noted that other designs of the engagement means 74, 76 are possible. For example, the engagement means 74, 76 could be formed as rough surfaces on respective end faces of the body nut 54 and the clutch 56, the respective end faces facing each other. Therefore, when the clutch 56 and the body nut 54 are engaged, frictional force between the engagement means 74, 76 is large enough to hinder a rotational movement of the body nut 54.

To ensure that a dispense operation is only possible by means of the dispensing mechanism 52, the clutch 56 is engaged by fully inserting the tank 16 into the housing 12.

This is achieved in the following manner. The tank 16 comprises at least one ramp feature 84 projecting away from a rear side of the tank 16. The ramp feature 84 comprises a ramp 86 helically inclined with regard to the longitudinal axis L. In the embodiment shown in Fig. 9a, the tank 16 comprises two such ramp features 84 arranged diametrically opposed at the rear side of the tank 16.

The clutch 56 also comprises at least one ramp feature 88 being complementary to the ramp feature 84 of the tank 16. In particular, the ramp feature 88 of the clutch 56 comprises a ramp 90 that is helically inclined with regard to the longitudinal axis L in a manner complementary to the ramp 86 of the tank 16.

The ramp feature 88 of the clutch 56 is formed on an end surface of the clutch 56 facing the tank 16. In the embodiment shown in Fig. 9b, the clutch 56 comprises two such ramp features 88 arranged diametrically opposed at the end surface of the clutch 56 facing the tank 16, when assembled.

Engagement of the clutch 56 is achieved by inserting the tank 16 into the housing 12 and rotationally twisting the tank 16 about the longitudinal axis L. During the twisting movement (arrow T in Fig. 10) of the tank 16, the ramp feature 84 of the tank 16 and the ramp feature 88 of the clutch 56 get into contact, such that, when the tank 16 is further twisted the ramp 86 of the tank 16 slides along the ramp 90 of the clutch 56, which ultimately results in an axial displacement of the clutch 56 and an engagement with the body nut 54 (arrow E in Fig. 10).

In the entirely assembled state, the outer most tips of the respective ramp features 84, 88 as seen in direction of the longitudinal axis L contact each other (see Fig. 10).

During engagement of the clutch 56, the clutch 56 is moved against a spring force of a spring 92 used to bias the clutch 56 away from the body nut 54 for disengagement.

In the embodiment shown in Figs. 6 to 8, the spring 92 is arranged between the blocking element 40 and a shoulder 94 formed at the clutch 56. However, the spring 92 may also bear against the inner wall of the housing 12 instead of the blocking element 40.

Furthermore, the spring 92 is arranged radially inwardly with respect to the guidance means 73 of the clutch 56 (see Figs. 7 and 8). This allows for the spring 92 to be securely held in place.

Engagement of the piston rod 50 with the plunger 58 of the tank 16 and dispense operation is achieved as follows.

The spring 60 pushes the piston rod 50 towards the front end of the dispenser, when the clutch 56 is disengaged and the body nut 54 is allowed to freely rotate. Upon insertion of the tank 16 into the housing 12, the piston rod 50 gets in contact with the plunger 58. Upon further insertion of the tank 16, the piston rod 50 is pushed into a direction opposite to the dispensing direction (towards the rear end of the dispenser 10), with the piston rod 50 still being in contact with the plunger 58.

The spring 60 is compressed and the spring force of the spring 60 ensures that the piston rod 50 and the plunger 58 remain in contact. This contact is still maintained when the clutch 56 and the body nut 54 are engaged, thereby allowing for an immediate dispensing operation. However, as the frictional force between the body nut 54 and the piston rod 50 is larger than the spring force of the spring 60, dispensing operation is only achieved upon operation of the dispense activator 22.

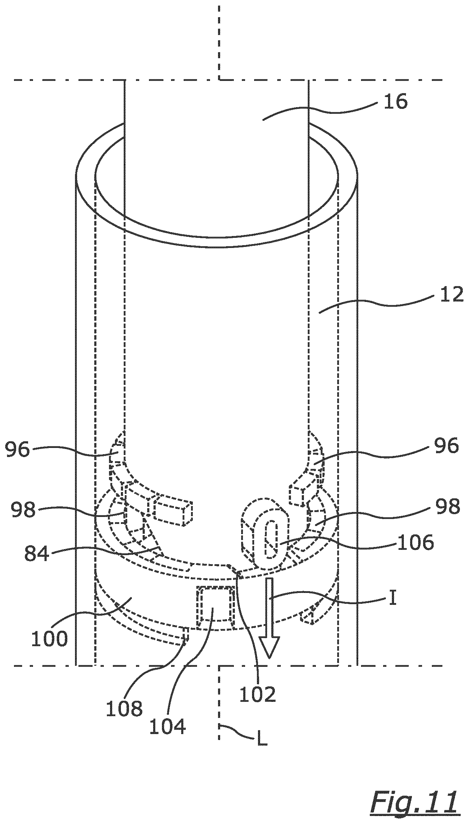

Now referring to Fig. 11, a further advantageous aspect related to keying or dedication of components of the dispenser 10 is described. Such kind of keying or dedication is desirable because the multi-use dispenser 10 may be used with various tanks 16 containing different materials, in particular different medications, adhesives, paints or the like. However, it needs to be ensured that the dosing is correctly set for each tank used.

Fig. 11 shows an exemplary dedication mechanism used to connect the correct tank 16 into the correct housing 12. However, the dedication mechanism described herewith is also applicable to any other part combination of the dispenser 10, including for example, but not limited to connections between the applicator tip 18 and/or the tank 16 and/or the housing 12 and/or the shielding 14.

In the embodiment shown in Fig. 11, the dedication mechanism is realized as first coding means 96 formed at an outer circumferential surface of the tank 16 and matching second coding means 98 formed on the inner wall of the housing 12, which in this case is the inner circumferential surface of the inner wall of the housing 12.

In the present embodiment, the first coding means 96 is formed by a number of protrusions and indentations of varying width, whereas the second coding means 98 is formed by corresponding number of indentations and protrusions with complementary width. Consequently, the tank 16 and the housing 12 can only be assembled if the first coding means 96 and the second coding means 98 match. In Fig. 11 the coding means 86, 98 are shown schematically as blocks.

The first and second coding means 96, 98 may also be realized in another way. By means of example, the first and second coding means 96, 98 could be realized by a typical magnetic pattern. It is to be understood that the magnetic pattern of the first and second coding means 96, 98 are complementary to each other.

As can be seen from Fig. 11, the tank 16 as well as the housing 12 each comprise a set of two identical coding means 96, 98. Within a set of coding means, a first coding means is configured in a point symmetrical manner with regard to a second coding means of the same set of coding means.

It is to be noted that such kind of sets of coding means could be also used for the connection between the other parts of the dispenser 10.

In order to allow for versatile applications, the second coding means 98 present in the housing 12 may formed on a ring 100 fixedly connected to inner circumferential surface of the housing 12. Upon the use of a designated tool, the ring 100 may be removed from the housing 12 and may be replaced by another ring 100 comprising other coding means 98. The dispenser 10 may then be used with another tank 16 comprising matching coding means 96.

Furthermore, the ring 100 forms a part of a bayonet type connecting mechanism and for this reason comprises a groove 102 in the inner circumferential surface of the ring 100. The groove 102 extends parallel to the longitudinal axis L. That is, the groove 102 extends parallel to a passage surrounded by the ring 100, with the passage configured to receive the tank 16.

The other part of the bayonet type connecting mechanism is formed by a circumferentially extending groove 108 and a notch 104, each formed in the inner circumferential surface of the housing 12. It is to be understood, that the circumferentially extending groove 108 and the notch 104 could be also formed in the ring 100, i.e. the entire bayonet type connecting mechanism is formed in the ring 100.

Assembly of the tank 16 in the housing 12 functions as follows.

If the first and second coding means 96, 98 match then a protrusion 106 formed on the outer circumferential surface of the tank 16 passes through the groove 102 of the ring (arrow I in Fig. 11). By twisting the tank 16, the protrusion 106 is guided in the channel 108 and ultimately comes at rest at the notch 104 in the inner surface of the housing 12. It is noted that the spring 92 axially biasing the clutch 56 to the front end also acts on the tank 16 via the respective ramp features 84, 88, thereby bringing the protrusion 106 of the tank 16 at rest at the notch 104 of the housing 12. In other words, the spring 92 supports in the assembly of tank 16 to the housing 12.

However, when the tank 16 is reversely twisted to remove the tank 16 from the housing 12, the spring 92 may also assist to eject the tank 16, when the protrusion 106 passes by the groove 102, thereby supporting the disassembly of the dispenser 10.

The protrusion 106 is preferably spring elastically deflectable in radial direction with respect to the longitudinal axis L. This allows for the protrusion 106 to snap fit into the notch 104 if the tank 16 is entirely inserted into the housing 12, thereby securely retaining the tank 16 in the housing 12.

Now referring to Figs. 12 and 13 an advantageous connection mechanism for the connection of the applicator tip 18 to the tank 16 is described.

As can be seen from Figs. 12 and 13, the applicator tip 18 comprises a protrusion 110 that upon connection to the tank is guided in a guide track 112 formed on the outlet side of the tank 16. The protrusion 110 and the guide track 112 together form a bayonet type connecting means.

The protrusion 110 is elastically deflectable in radial direction with regard to the longitudinal axis L.

It is to be noted that the protrusion may also be formed at the tank 16 and the guide track may be formed at the applicator tip 18.

It is further noted that the connection mechanism described herein with regard to the applicator tip 18 and the tank 16 is also applicable for a connection between the applicator tip 18 and the housing 12 and/or the shielding 14.

As can be seen from Fig. 13, the guide track 112 comprises a first portion 112a that is helically inclined and comprises an extension component that extends into the longitudinal direction. Such kind of orientation of the first portion 112a forces the applicator tip 18 onto the tank 16, when attached, and pushes the applicator tip 18 of the tank 16, when detached.

A second portion 112b of the guide track 112 extends into a direction circumferentially surrounding the longitudinal axis L. The first portion 112a and the second portion 112b transition into each other.

Furthermore, a detent 114 is formed in the guide track 112 that prevents unwanted detachment of the applicator tip 18 from the tank 16. However, upon applying a predefined force, the protrusion 110 can be moved over and past the detent 114. The force is a force that is typically applied by a user manually operating of the dispenser 10, i.e. the applicator tip 18 can be attached and detached by hand using a typical hand strength. Preferably, the applicator tip 18 can be attached to or detached from the tank 16 by single-hand operation.

In particular, the detent 114 hinders that the protrusion 110 guided in the circumferentially extending second portion 112b is moved towards the first portion 112a such that the applicator tip 18 is accidentally detached from the tank 16 during rotational movement of the applicator tip 18.

In the embodiment shown in Fig. 13, the detent 114 is formed in the of the second portion 112b and defines a starting point of the second portion 112b. In particular, the detent 114 is formed in the second portion 112b, where the first portion 112a transitions into the second portion 112b.

Furthermore, the second portion 112b ends at a stopping member 116. The stopping member 116 separates the end of the second portion 112b from the first portion 112a of the guide track 112.

As can be seen from Fig. 13 the part of the stopping member 116 facing the first portion 112a lies flush with the first portion 112a and has the same inclination with regard to the longitudinal axis L like the first portion 112a.

The detent 114 and the stopping member 116 define rotational angle of the second portion 112b of at least 270°, preferably of at least 280° and more preferably of at least 300°. That is, the applicator tip 18 can be rotated relative to the tank 16 by a rotational angle of at least 270°, preferably of at least 280° and more preferably of at least 300°. This is desirable if the applicator tip 18 comprises a bent cannula 20. The cannula 20 can then be rotated in a desired position for application of a material. Yet the dispense activator 22 remains in a position which is comfortable for the user.

However, to keep the applicator tip 18 in a desired rotational position, at least one of the tank 16 and the applicator tip 18 may comprise at least one interference means 124.

The interference means 124 causes an interference fit between the tank 16 and the applicator tip 18, in particular when fully assembled.

In the embodiment shown in Fig. 12 the interference means 124 is formed by three splines 126 protruding radially inwardly from an inner surface of the applicator tip 18 and extending in axial direction with regard to the longitudinal axis L. The number of the splines 126 may vary, i.e. to be less than three or more than three, such as one spline 126, two splines 126, four splines 126 or five or more splines 126.

An interference means 124 may also be provided on an outer circumferential surface of a portion of the tank 16 interacting with the applicator tip 18. In this case, the interference means 124 may be formed for example by portions radially protruding from the tank 16.

Additionally or alternatively, an interference fit can also be created by the interaction of the protrusion 110 with the second portion 112b of the guide track 112. This can be achieved for example if a width of the protrusion 110 as seen in the longitudinal axis L is slightly larger than a width of the second portion 122b of the guide track 112 as also seen in the longitudinal axis L.

In this context, as can be seen from Fig. 13, the width of the first portion 112a as seen in the circumferential direction that is larger than a width of the second portion 112b as seen in the longitudinal direction L. To ensure safe guidance, the protrusion 110 may be sized as to basically correspond to the dimensions of the first and second portions 112a, 112b of the guide track 112.

According to another embodiment basically corresponding to the one just described, but not shown, the detent 114 is formed in the first portion 112a of the guide track 112 instead of the second portion 112b. In particular, in the end part of the first portion 112a that transitions into the second portion 112b. Furthermore, the detent 114 of this embodiment may lie flush with the second portion 112b.

Such kind of configuration allows for a rotational angle of nearly 360°, which is only limited by the circumferential dimension of the stopping member 116. Hence, the applicator tip 18 can be rotated to an even larger extent.

According to still another embodiment not shown, the detent 114 may also be formed in that region in which the first portion 112a transitions into the second portion 112b. It is to be understood, that additionally at least one further detent 114 may be also formed in at least one of the first and second portions 112a, 112b.

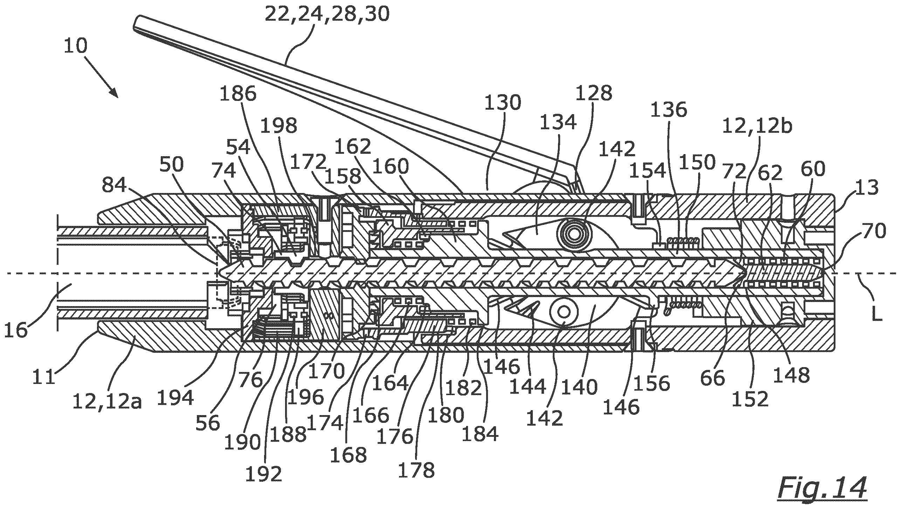

Now with reference to Figs. 14 to 16 a further advantageous embodiment (second embodiment) of a dispenser 10 is described. Unless described to the contrary, the functioning of the dispenser 10 of the further embodiment corresponds to the functioning of the dispenser 10 described beforehand. In the following, the dispenser 10 of the second embodiment is described with continued reference to the dispenser 10 of the first embodiment and emphasize is put on the basic differences.

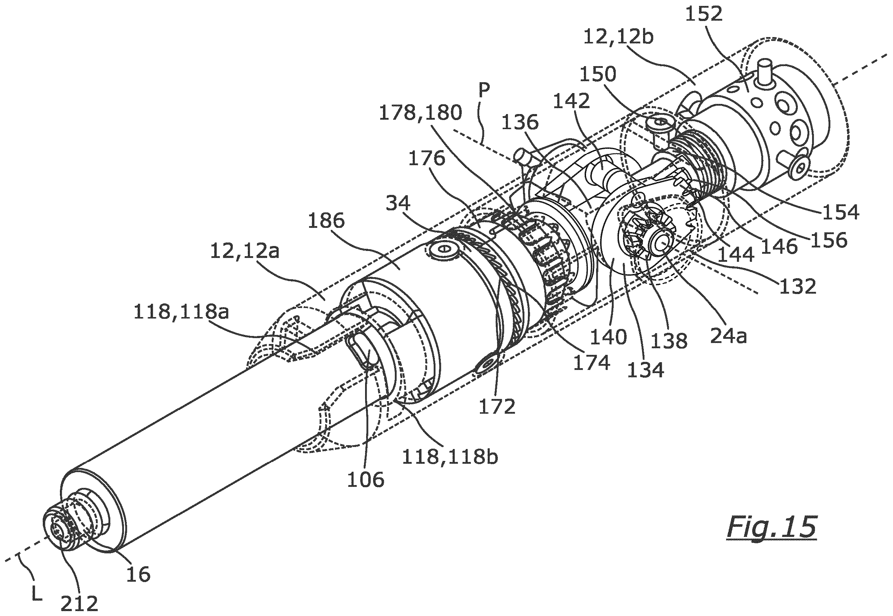

In Fig. 14 and 15 the dispenser 10 of the second embodiment is depicted without any shielding 14. However, the dispenser 10 may comprise a shielding 14 as described above with regard to the first embodiment of the dispenser 10.

Furthermore, in the dispenser 10 of the further embodiment, the housing 12 of the dispenser 10 is made of multiple parts and comprises a front housing part 12a and a rear housing part 12b which facilitates the assembly of the dispenser 10 and its parts. It is noted that the housing 12 of the dispenser 10 according to the second embodiment may be also formed as a single piece.

In order to initiate dispense action, the dispenser 10 of the second embodiment comprises a single-arm lever 24. The lever 24 has the function of a dispense activator 22, wherein the arm of the lever 24 acts as a push arm 28 or push button 30 activatable by a user for dispense action.

The lever 24 is pivotable about a pivot axis P aligned at least substantially perpendicular to the longitudinal axis L of the dispenser 10.

On its base 128 the lever 24 is bifurcated, such that the housing 12 is received between two tines 130 of the bifurcated base 128.

Furthermore, as can be seen best from Fig. 15, the side wall of the housing 12 comprises two diametrically opposed circular break throughs 132 through which the lever 24 can interact with a transmission means 134 used for transferring a rotational movement of the lever 24 onto a drive tube 136 rotationally accommodated in the housing 12.

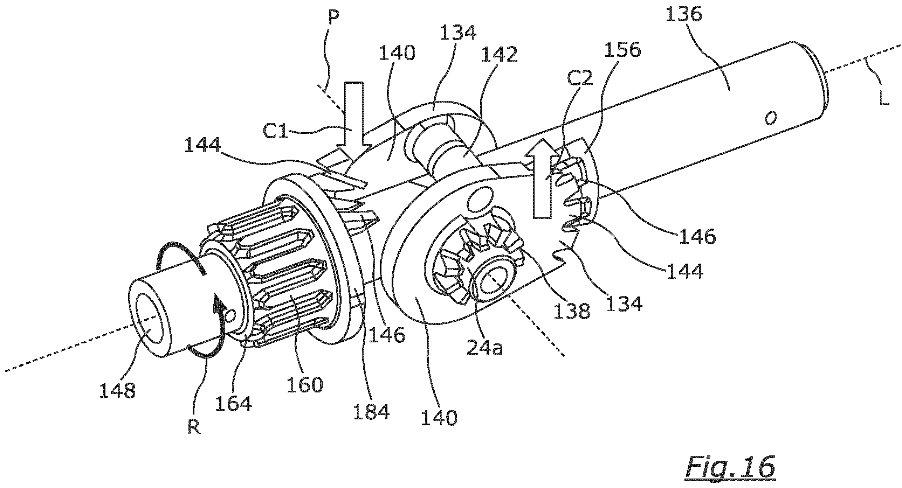

For this purpose, the lever 24 comprises on respective inner sides of the tines 130 toothed engagement means (not visible) that interact with corresponding complementary toothed engagement means 138 formed on respective outward facing sides of respective drive plates 140 of the transmission means 134 (Fig. 16). This rotationally fixes the lever 24 to the transmission means 134 to ensure that a rotation of the lever 24 about the pivot axis P results in a corresponding rotation of the transmission means 134 about the pivot axis P.

As can be seen from Fig. 16, the toothed engagement means 138 of the transmission means 134 are arranged radially about the pivot axis P and define a fulcrum 24a of the lever 24.

The lever 24 is fixed to the transmission means 134 by a screw type or a bolt type connection with the screw or bolt coaxially aligned with the pivot axis P. However, the lever 24 may be also connected to the transmission means 134 by other connection types, such as for example using an adhesive.

In the present embodiment, the transmission means 134 comprises two drive plates 140 capable of a counter directed rotational movement about the pivot axis P, as is depicted by arrows C1 and C2 in Fig. 16. The counter directed rotational movement of the drive plates 140 is induced by a rotation of the lever 24 about the pivot axis P.

In order to enhance torsional stiffness of the transmission means 134, the drive plates 140 are connected to each other by a pair of tubular hollow shafts 142 extending at least substantially parallel to the pivot axis P. The shafts 142 are respectively radially off-set with regard to the pivot axis P in order to avoid collision with the drive tube during dispense operation.

Furthermore, each of the drive plates 140 comprise on an inner side, i.e. a side opposite to the side comprising the engagement means 138 for the lever 24, a bevel gear 144.

As can be seen from Fig. 16 the respective bevel gears 144 face in opposite directions. In particular, the teeth of the respective bevel gears 144 face in opposite axial directions, i.e. the teeth of one bevel gear 144 face towards the front end 11 of the dispenser 10, whereas the teeth of the other bevel gear 144 face towards the rear end 13 of the dispenser 10.

The bevel gears 144 of the transmission means 134 mesh with corresponding bevel gears 146 formed on an outer side wall of a drive tube 136. The bevel gears 146 of the drive tube 136 are formed on oppositely averted sides of the outer side surface of the side wall of the drive tube 136. Furthermore, like the bevel gears 144 of the transmission means 134, the bevel gears 146 of the drive tube 136 face in opposite directions. In particular, in the present embodiment, the teeth of the bevel gears 146 face in opposite axial directions, i.e. the teeth of one bevel gear 146 face towards the front end 11 of the dispenser 10, whereas the teeth of the other bevel gear 146 face towards the rear end 13 of the dispenser 10.

Hence, when the drive plates 140 are rotated by the lever 24, the counter directed bevel gears 144 formed thereon rotate in opposite directions, thereby driving the counter directed bevel gears 146 formed on the drive tube 136 in opposite directions, which ultimately results in a rotation of the drive tube 136 in a first rotation direction (arrow R in Fig. 16).

**In** principle, a rotational movement can be also transmitted onto the drive tube 136 by using just one drive plate 140 configured in a manner as described above. However, using two drive plates 140 on opposite sides of the drive tube 136 allows for a more balanced force transmission onto the drive tube 136.

It is noted that a rotation of the drive tube 136 taken alone, i.e. without further parts of the dispenser 10, does not rotate the piston rod 50, as the drive tube 136 receives the piston rod 50 in a slideable non-engaged manner in a passage 148 axially extending through the drive tube 136.

As the piston rod 50 does not rotate with the drive tube 136, the drive tube 136 is allowed to rotate back into its initial position for further dispensing action without rotating the piston rod 50 backwards.

Backwards rotation of the drive tube 136 in a second rotation direction opposite to the first rotation direction is initiated by the spring force of a return spring 150. In fact, as the drive tube 136 is rotated in the first rotation direction, the return spring 150 is loaded. Thus, when the lever 24 is released the return spring 150 unloads and rotates the drive tube 136 back into its initial position. Due to the interaction of the drive tube 136 with the lever 24 via the transmission means 134, the lever 24 is also erected for further dispense action.

In the present embodiment, the return spring 150 is a torsional spring that circumferentially surrounds the drive tube 136.

It is noted that additionally or supplementary, the lever 24 can be pushed against the spring force of another return spring, such as a compression spring radially arranged between the outer side of the housing 12 and the inner side of the lever 24. As the lever 24 is released such kind of spring tends to expand, thereby erecting the lever 24 into its initial position. Again, as the lever 24 interacts with the drive tube 136 via the transmission means 134, the return rotation of the lever 24 into its initial position results in a rotation of the drive tube 136 in a direction opposite to the first rotation direction, i.e. the second rotation direction.

Furthermore, it is also possible to use a compression spring that is expanded during pushing of the lever 24 and that retracts as the lever 24 is released to rotate the lever 24 back into its initial position and also to rotate the drive tube 136 backwards.

In the present embodiment, the spring force of the torsional return spring 150 is adjustable. This is achieved by a preload means 152 inserted into the housing from its rear end. The preload means 152 comprises a spiral groove extending about the longitudinal axis L and receiving the rear end of the torsional return spring 150. The front end of the torsional return spring 150 bears against a washer 154 which in turn bears against a collar 156 formed by the rearwards facing bevel gear 146 of the drive tube 136. By turning the preload means 152 about the longitudinal axis L, the torsional return spring 150 is expanded more or less, thereby changing the return force of the torsional return spring 150. A desired spring force can be fixed by tightening the preload means 152 to the housing, for example by using a screw.

It is noted that in the present embodiment, the preload means 152 closes an axial rear end opening of the drive tube passage 148.

However, it is also possible to produce the dispenser without the preload means 152, i.e. without the possibility to adjust the spring force of the return spring 150. The return spring 150 has then a predefined fixed return force. In this case the axial rear end opening of the drive tube passage 148 may be closed by an inner end surface 64 of the rear end of the housing 12.

Furthermore, it is also possible that the axial rear end of the passage 148 of the drive tube 136 may be closed instead of comprising an open axial rear end.

In either case, closing the rear end of the drive tube's passage 148 prevents that the piston rod 50 slides out of the drive tube 136 from its rear end.

In contrast to the rear end of the drive tube 136, the axial front end of the drive tube 136 is open which allows the piston rod 50 to move axially out of the passage 148 of the drive tube 136 towards the dispenser's front end 11 for dispense action.

Axial advancement of the piston rod 50 is achieved likewise to the axial advancement of the piston rod 50 as described with regard to the first embodiment, i.e. by a rotational movement of a drive nut 34 in combination with a threaded engagement with a rotationally fixed body nut 54.

In particular, in the second embodiment of the dispenser 10, rotational movement of the drive tube 136 is transferred onto the drive nut 34 by means of a drive ratchet 158.

The drive ratchet radially 158 surrounds the drive tube 136. Furthermore, the drive ratchet 158 is rotationally locked with regard to the drive tube 136, but allowed to axially move with regard to the drive tube 136. In the present embodiment this is achieved by a splined connection between the drive ratchet 158 and the drive tube 136 with splines 160 formed on an outer surface of the drive tube 136 and splines 162 formed on an inner side of the drive ratchet 158, the splines 160, 162 extending at least substantially parallel to the longitudinal axis L.

The splines 160 of the drive tube 136 are closed at the rear end, thereby limiting an axial travel of the drive ratchet 158 towards the rear end.

Furthermore, the splined features 160 of the drive tube 136 are formed on a shoulder 164 radially protruding from the side wall of the drive tube 136.

The axial front end of the shoulder 164 acts as a counter bearing for a drive ratchet spring 166 that biases the drive ratchet 158 towards the front end 11 of the dispenser 10 against the drive nut 34. This allows for ratchet teeth 168 formed on an axial front end of the drive ratchet 158 to engage with corresponding ratchet teeth 170 formed on an axial rear end of the drive nut 34. It is mentioned that the drive ratchet spring 166 extends axially between the axial front end of the shoulder 164 formed on the drive tube 136 and the drive nut 34.

The drive ratchet spring 166 is configured as a compression spring and circumferentially surrounds the drive tube 136.

The ratchet teeth 168, 170 of the drive ratchet 158 and the drive nut 34 interact in a manner as to engage with each other to rotate the drive nut 34 in the first direction as the drive tube 136 is rotated in the first direction. That is, the drive nut 34 and the drive tube 136 rotate together in the first direction. In a rearward rotation, i.e. in a second rotation direction opposite to the first rotation direction, the ratchet teeth 168, 170 slide past each other without any rotational transmission. The drive ratchet 158 is then axially moved against the force of the drive ratchet spring 166, thereby allowing the ratchet teeth 168, 170 passing by each other.

The drive nut 34 comprises a further set of ratchet teeth 172 interacting with ratchet teeth 174 formed on a counter ratchet 176.

The counter ratchet 176 like the drive ratchet 158 is axially movable. However, the counter ratchet 176 is rotationally fixed with regard to the dispenser housing 12. This is achieved by a splined connection between the counter ratchet 176 and the housing 12 with respective splines 178, 180 of the counter ratchet 176 and the housing 12 respectively extending in axial direction. In particular, the splines 178 of the counter ratchet 176 are formed on an outer surface of the counter ratchet 176, whereas the splines 180 of the housing 12 formed on the inner side of the side wall of the housing 12.

Furthermore, the counter ratchet 176 at least in sections radially surrounds the drive ratchet 158. Consequently, the ratchet teeth 172 of the drive nut 34 interacting with the counter ratchet 176 are off-set radially outwardly with regard to the ratchet teeth 170 of the drive nut 34 interacting with the drive ratchet 158 (Fig. 14). Furthermore, the ratchet teeth 174 of the counter ratchet 176 are formed on an axial front side of the counter ratchet 176, whereas the corresponding ratchet teeth 172 of the drive nut 34 are formed on the axial rear side of the drive nut 34.

In order to engage the ratchet teeth 172, 174 of the drive nut 34 and the counter ratchet 176, the counter ratchet 176 is biased towards the drive nut 34 by a counter ratchet spring 182. For this purpose, the counter ratchet spring 182 bears against the counter ratchet 176 on one side and on the other axial side against a collar 184 formed on the drive tube 136. The collar 184 radially extends with regard to the splines 160 formed on the drive tube 136. However, the counter ratchet spring 182 may also bear against a collar protruding radially inwardly from the inner side of the side wall of the housing 12.

The counter ratchet spring 182 is formed as a compression spring.

The counter ratchet 176 blocks a rotation of the drive nut 34 in the second direction opposite to the first direction during resetting of the piston rod 50, i.e. a movement of the piston rod 50 towards the rear end, for instance as the tank 16 is inserted into the dispenser 10.

The blocking direction of the counter ratchet 176 and the drive ratchet 158 are effective in the same direction.

Furthermore, like in the dispenser 10 of the first embodiment, in the dispenser 10 of this embodiment the body nut 54 is rotationally locked, when the tank 16 is fully inserted into the dispenser, as described above with regard to the first embodiment.

Rotational locking of the body nut 54 is achieved by means of a clutch 56, in a similar manner as described with regard to the first embodiment.

In the present embodiment, the clutch 56 is axially movable and in form fit engagement with an insert 186 inserted into the housing 12, the insert 186 rotationally fixed with respect to the housing 12 and the insert 186 circumferentially surrounding the clutch 56.

In particular, the clutch 56 comprises axially extending splines 188 on an outer surface of the clutch 56 interacting with corresponding axially extending splines 190 formed in an inner surface of the insert 186. The clutch 56 can therefore be moved in axial direction.

It is noted that the dispenser 10 can also be formed without the insert 186. In this case, the splines interacting with the splines 188 of the clutch 56 are formed on an inner side wall of the housing 12. In this regard, the clutch 56 is axially moved against the spring force of a clutch spring 192 from the disengaged state into the engaged state. That is the clutch spring 192 disengages the clutch 56 from the body nut 54 as the tank 16 is removed, thus allowing the body nut 54 to freely rotate.

However, in the engaged state of the clutch 56, engagement means 76 of the clutch 56 engage with corresponding engagement means 74 of the body nut 54, in a dog clutch like manner.

As mentioned above, the clutch 56 is engaged by a full axial insertion of the tank 16 into the dispenser. This axial movement of the tank 16 is transmitted onto the clutch 56 by a reactor plate 194 axially arranged between the tank 16 and the clutch 56.

In a disengaged state of the clutch 56, the body nut 54 is allowed to freely rotate. The piston rod 50 can then be pushed rearwards to the rear end 13 of the dispenser 10 for further dispense action.

Furthermore, the insert 186 is also used to prevent an axial movement of the drive nut 34 towards the front end 11. For this purpose, the insert 186 forms a radially inwardly projecting neck 196. The neck 196 is formed at the rear side of the insert 186 and acts as a counter bearing for the drive nut 34 such that the drive nut 34 bears against the rear side of the insert 186. That is the rear side of the insert 186 hinders that the drive nut 34 from being axially moved towards the front end 11 of the dispenser 10, for instance by the force of the drive ratchet spring 166 and/or the counter ratchet spring 182.

The insert 186 and its neck 196 comprise an axial passage 198 for the piston rod 50.

It is noted that a corresponding neck can be formed on the housing 12 such as to extend radially inwardly from an inner side of the housing's side wall if the dispenser 10 does not comprise the insert 186.

Like in the dispenser 10 of the first embodiment, the piston rod 50 is pushed rearwards against the spring force of a spring 60 during insertion of the tank. The spring force of the spring 60 therefore urges the piston rod 50 towards the front end, thereby enabling the piston rod 50 to be engaged with a plunger 58 (Fig. 4) inserted in the tank 16. This allows for direct dispensing from the tank 16 or at least after only a few operational clicks of the lever 24 even if the tank 16 is installed or reinstalled that was partly emptied or originally only partly filled.

The spring 60 acts on the piston rod 50, with the spring force of the spring 60 being selected such that the piston rod 50 is urged in the dispensing direction towards the plunger 58 of the tank 16, thereby making the dispenser 10 directly ready to use after insertion of the tank 16.

Like in the dispenser 10 of the first embodiment, the spring 60 of the dispenser of this embodiment acts with one end on the piston rod 50 by means of a transmitter element 62, whereas the other end of the spring 60 bears against the preload means 152.

However, the other end may also bear against the housing 12, in particular an inner end surface 64 of the housing 12, as shown in Figs. 4 and 7, if the dispenser does not comprise the preload means 152.

Furthermore, the spring 60 may bear against a closed rear end of the passage 148, if the rear end of the drive tube's passage 148 is closed.

The spring 60 as well as the transmitter element 62 are both received in the passage 148 of the drive tube 136, as can be seen best from Fig. 14. This allows for a more compact setup of the dispenser 10.

The transmitter element 62 of this embodiment is formed as a pin with a T-like shape as seen in the longitudinal sectional view of Fig. 14.

Like the transmitter element 62 of the dispenser 10 of the first embodiment, the transmitter element 62 of the dispenser 10 of this embodiment has a cavity 66 to receive the rear end of the piston rod 50 (Fig. 14). The cavity 66 has the shape of a hollow cone. This reduces frictional forces with regard to the piston rod 50, in particular if the piston rod 50 also has a cone shaped rear end interacting with the cavity 66. In this regard it is noted that the cone shaped rear end of the piston rod 50 may have a smaller opening angle as the cone shaped cavity 66, as can be seen for example in Figs. 4, 6 and 14.

The transmitter element 62 also acts as a stopper for the rearwards travel of the piston rod 50, as the rear tip of the pin opposite to the cavity 66 contacts the preload means 152 or inner rear side wall 64 of the housing 12, when the piston rod 50 is fully retracted.

Now turning to Figs. 17 to 21 further embodiments of an applicator tip 18 are described.

Each of the applicator tips 18 shown in Figs. 17 to 21 extends between a rear end and a front end along an axial direction, i.e. a longitudinal axis L of the applicator tip 18. The front end comprises a closed front wall 200 that transitions into a side wall 202 circumferentially extending about the axial direction L.

The front wall 200 and the side wall 202 define a cavity 204 configured to receive a further component of the dispensing system, such as for instance the tank 16, the housing 12 or the shielding 14, through the open rear end of the applicator tip 18.

In the following the applicator tip 18 will be described as an applicator tip 18 connectable or connected to a tank 16. However, the same description also applies to a connection of the applicator tip 18 to the housing 12 and/or the shielding 14.

The side wall 202 is radially deflectable with regard to the axial direction L and further comprises a connection member 206 formed on an inner side of the side wall 202.

In the embodiments shown on Figs. 17 to 21 the connection member 206 is formed as a part of a snap fit connection type.

Such kind of snap fit connection can be achieved by the side wall 202 being formed of a spring elastic material and/or by choosing the side wall thickness to allow for a spring elastic deflection of the side wall 202. It is noted that by choosing the side wall thickness to allow for spring elastic deflection thereof, the side wall 202 can be either formed of a spring elastic material or of a rigid material, as long as the side wall of the applicator tip 18 can be spring elastically deflected.

It is noted, the connection member 206 of the applicator tips 18 shown in Figs. 17 to 21 may be also formed as a part of a bayonet type or a screw type connection assembly. In particular, the connection member 206 may be also formed in a manner as described with regard to Figs. 12 and 13.

Now turning back to Figs. 17 to 21, the connection member 206 of the applicator tips 18 shown in these figures is formed as a protrusion that entirely extends on the inner side wall in circumferential direction in a ring like manner. It is noted that the connection member 206 may also be formed as a recess at least partially extending in circumferential direction.

The corresponding connection member 208 of the tank 16 is formed fully complementary to the connection member 206 of the applicator tip 18, i.e. as a ring like recess.

Furthermore, the applicator tip 18 may comprise a plug 210 that axially extends from an inner side of the front wall towards the rear end of the applicator tip.

In a connected state, in which the tank 16 is received in the cavity 204 of the applicator tip 18, the plug 210 extends into an outlet opening 212 of the tank 16, such that an outer surface of the plug 210 tightly bears against an inner surface of the outlet opening 212, as shown for instance in Fig. 18.

Furthermore, if the is front wall 200 as well as the plug 210 are closed, leakage from the tank 16 is prevented if the plug 210 bears against the tank's opening 212, in particular if connected in a fluid tight manner. Even further, if the plug 210 bears against the opening 212 of the tank 16 in a gas tight manner, gas transfer is hindered or at least reduced such that premature degradation of the material stored in the tank 16 is prevented. In either case, the applicator tip 18 acts as a closure cap.

A fluid tight and/or gas tight applicator tip 18 is achieved if the plug 210 is formed as a cone or truncated cone, as shown for instance in Figs 17, 18 or 20. However, for the same purpose the plug 210 may also have other shapes, such as a cylindrical shape, as long as the side wall fluid tightly and/or gas tightly bears against the opening 212 of the tank 16.

Furthermore, as becomes apparent from Fig. 19, instead of being closed, the plug 210 and the front wall 200 of the applicator tip 18 may comprise a passage 214 allowing for discharging of a material stored in the tank 16.

Furthermore, generally speaking, the passage 214 axially extending through the plug 210 and the front wall may 200 receive a cannula 20 formed separately with regard to the applicator tip 18. However, in the embodiment shown in Fig. 19, the passage 214 is formed by the cannula 20 being an integral part of the applicator tip 18. That is, the cannula 20 and the applicator tip 18 are formed as a single piece. In this regard it is noted that the cannula 20 may be a straight cannula, a bendable cannula or a bent cannula as has been detailed above.

Even further, the applicator tip 18 can be connected to the tank 16 by a predetermined breaking means 216, thereby forming a storage system. The predetermined breaking means 216 allows for the detachment of the applicator tip 18 from the tank 16 upon applying a predetermined breaking force.

In the present embodiment, the predetermined breaking means 216 is formed between the tank 16 and a side of the front wall 200 that when the tank 16 is received in the cavity 204 of the applicator tip 18 forms an outer side of the applicator tip 18. That is, after breaking the applicator tip 18 away from the tank 16, the applicator tip 18 can be flipped over by a rotation of about 180° about an axis perpendicular to the longitudinal axis L, i.e. the axial direction of the applicator tip 18, and can then be used as a closure cap for the tank 16. However, it is noted that the front wall 200 of the applicator tip 18 can be configured to be pierceable, to form an outlet passage 214 to allow the applicator tip 18 to be used for discharge action.

In the embodiment shown in Fig. 17, the longitudinal axis L of the applicator tip 18 and the longitudinal axis L of the tank 16 are coaxially aligned, when connected by the predetermined breaking means 216. Furthermore, in Fig. 16, after flipping the applicator tip 18 over and reattaching the applicator tip 18 to the tank 16 by means of the connection assembly, the longitudinal axis L of the applicator tip 18 and the longitudinal axis L of the tank 16 are again coaxially aligned.

It is further noted that, according to another embodiment, the predetermined breaking means 216 can be also formed between an inner side of the front wall 200 and the tank 16. In such configuration no flipping over of the applicator tip 18 is needed. The applicator tip 18 can be just twisted against the tank 16 to break off the predetermined breaking means 216. Further twisting of the applicator tip 18 can turn the applicator tip 18 in a position for engagement with the tank 16 as described above. It is noted that such kind of applicator tip 18 can also be pierced to form an outlet passage 214 for discharge action.

In either way, the predetermined breaking means 216 can form a seal that closes the outlet opening 212 of the tank 16, when the applicator tip 18 is connected to the tank 16 by the predetermined breaking means 216. For instance, the predetermined breaking means 216 may circumferentially surround the outlet opening 212 of the tank 16 and together with the front wall 200 of the applicator tip 18 sealingly closes the outlet opening 212 of the tank 16.

Hence, the tank 16 is provided with the appropriate applicator tip 18, wherein the opening 212 of the tank 16 is closed as long as the applicator tip 18 is not detached by breaking off the predetermined breaking means 216. In other words, the tank 16 can be provided with the appropriate applicator tip 18 and at the same time ensures fluid tight and/or gas tight closure of the tank 16 as long as the applicator tip 18 is connected to the tank 16 by the predetermined breaking means 216.

It is to be understood that the predetermined breaking means 216 breaks such that when the applicator tip 18 is reattached to the tank 16 by means of the connection assembly comprising the connection members 206, 208 the applicator tip 18 tightly sits on the tank 16, without the predetermined breaking means 216 forming any substantial gap between the to the front wall 200 of the applicator tip 18 and the front end of the tank 16. However, if the plug 210 is sufficiently long enough to tightly engage with the outlet opening 212 of the tank 16, then residues of the predetermined breaking means 216 may remain on the front end of the tank 16.

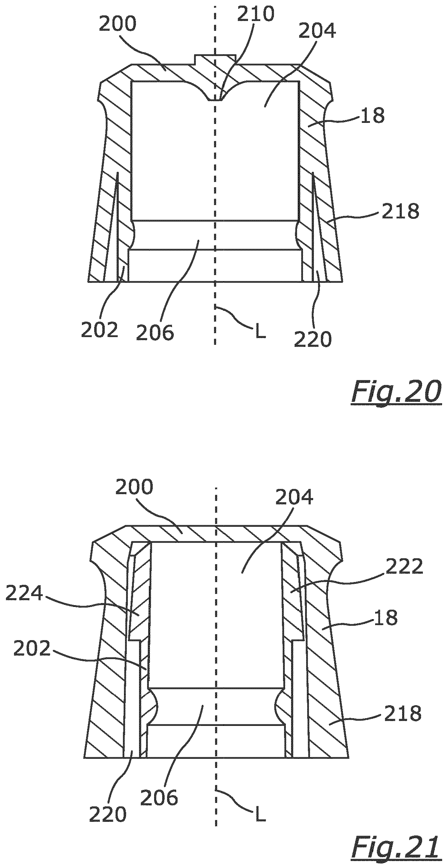

Fig. 20 and 21 show applicator tips 18 in which the elastically deflectable side wall 202 is surrounded by a further outer shell wall 218.

The shell wall 218 entirely surrounds the flexible side wall 202 in circumferential direction.

Furthermore, the shell wall 218 is more rigid than the side wall 202. In the present embodiment this is achieved by providing a wall thickness of the shell wall 218 thicker than the side wall 202. However, for the same purpose, the shell wall 218 may additionally or supplementary be formed of a material more rigid than a material of the side wall 202.

The shell wall 218 is used to avoid unwanted detachment of the applicator tip 18 as the shell wall 218 is less easy deflectable in comparison to the side wall 202 comprising the connection member 206.

As can be seen from Figs 20 and 21, a radial gap 220 is formed between the side wall 202 and the shell wall 218 thus allowing for a radial deflection of the side wall 202 during attachment or detachment of the applicator tip 18.

The radial gap 220 is formed by flaring the shell wall 218 towards the rear end of the applicator tip 18.

Furthermore, in the embodiment shown in Fig. 20 the side wall 202 and the shell wall 218 both respectively emanate from the front wall 200. However, depending on the application, the shell wall 218 can also emanate from the side wall 202.

In Fig. 21, the side wall 202 is formed on an insert 222 inserted into the cavity 204 of the applicator tip 18 and wherein the shell wall 218 emanates from the front wall 200. The side wall 202 in this embodiment is formed as a tubular hollow cylinder.

The side wall 202 and the shell wall 218 of the embodiment shown in Fig. 21 are connected by connection means 224 that in the present embodiment are formed as snap in connection means 224. Such kind of connection is beneficial due to the spring elastic configuration of the side wall 202. However, the side wall 202 can be also connected to the shell wall 218 by other connection types, for instance using an adhesive.

When the side wall 202 and the shell wall 218 of the applicator tip 18 of Fig. 21 are assembled, the shell wall 218 and the side wall 202 like in the embodiment shown in Fig. 20 both emanate from the front wall 200.

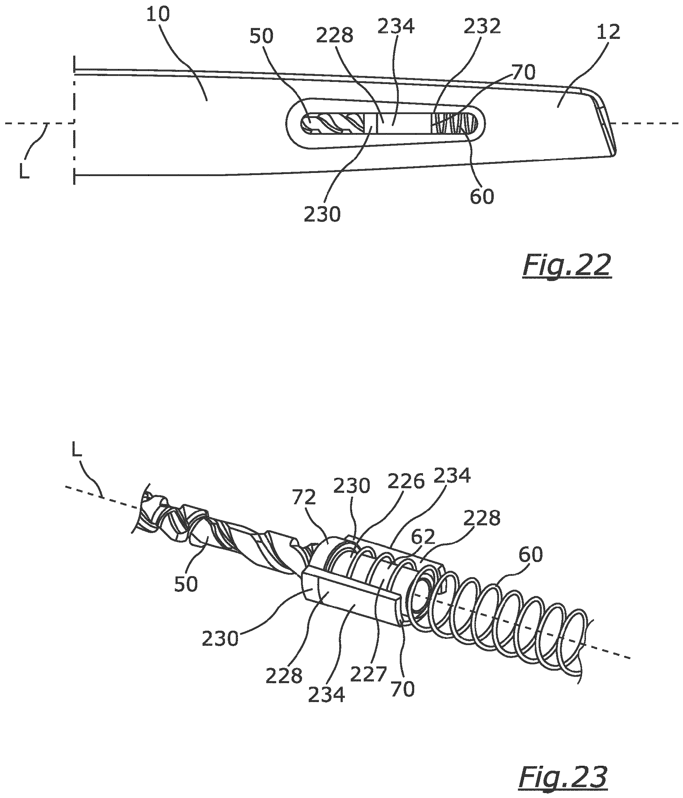

With reference to Figs. 22 and 23 an indicator means 226 is described. The indicator means 226 can be used in a dispenser 10 as described in the foregoing with regard to the first and second embodiments of a dispenser 10.

In particular, the indicator means 226 comprises a similar set up as the transmitter element 62 as described above. That is, the above described transmitter element 62 can be modified to form the indicator means 226 as described in the following. In particular, the indicator means 226 and the transmitter element 62 may be the same part.

In the embodiments shown in Fig. 22 and 23, the indicator means 226 like the transmitter element 62 moves together with the piston rod 50 in direction of the longitudinal axis L. This is achieved by a spring 60, such as the auto-prime spring, that bears with on one side on an inner rear end surface 64 of the housing 12 and that bears with another end against a collar 72 formed on the indicator means 226. The collar 72 radially protrudes from a side wall of a main body 227 of the indicator means 226.

As shown in Fig. 23, the main body 227 is formed as a cylindrical part. However, the main body 227 can also formed in a manner like the guidance structure 68 described above. It is further noted that the main body 227 can also have other shapes. In particular, the main body 227 may be pin like.

The main body 227 of the indicator means 226 is radially surrounded by the spring 60.

The spring 60 urges the indicator means 226 against the piston rod 50 in a manner as like the transmitter element 62 is pushed against the piston rod 50. The indicator means 226 hence moves together with the piston rod 50 in direction of the longitudinal axis L.

During forward travel of the piston rod 50, the spring 60 is allowed to expand thereby keeping the indicator means 226 in intimate contact with the piston rod 50. During a rearward movement of the piston rod 50, the spring 60 is compressed still keeping the indicator means 226 and the piston rod 50 in intimate contact. Hence, the indicator means 226 is capable of indicating an axial position of the piston rod 50 relative to the housing 12 of the dispenser 10. In this regard it is noted that an axial position of the piston rod 50 also correlates to how far the piston rod 50 has been moved into a tank 16 attached to the dispenser 10. Hence, the indicator means 226 is also indicative for the material stored in the tank 16.

In order to securely receive the piston rod 50 at the indicator means 226, the indicator means 226 like the transmitter element 62 may comprise a cone shaped cavity 66 formed on the front end of the indicator means 226 and that receives the cone shaped rear end of the piston rod 50.

The piston rod 50 can rotate relative to the indicator means 226, with the cone shaped rear end of the piston rod 50 and the cone shaped cavity 66 allowing reduced friction between the piston rod 50 and the indicator means 226. Furthermore, the cone shaped connection allows for a secure assembly of the piston rod 50 and the indicator means 226.

Furthermore, the indicator means 226 is rotationally fixed with regard to the housing 12 and/or the piston rod 50, while the piston rod 50 is allowed to rotate relative to the housing 12.

Rotational fixation of the indicator means 226 is achieved by the indicator means 226 comprising at least one guidance means 228 axially guided by at least one complementary guidance means formed at the housing (not shown).

In the present embodiment, the guidance means 228 of the indicator means 226 is formed by two protrusions 230 present on diametrically opposite outer circumferential sides of the collar 72. As can be seen from Fig. 23 each protrusion 230 axially extends from the collar 72 towards a rear end 70 of the indicator means 226 opposite to the front end.

The protrusions 230 of the indicator means 226 are respectively guided in corresponding grooves formed on the inner side of the side wall of the housing 12. The grooves extend parallel in direction of the longitudinal axis L.

It is to be understood that at least one of the guidance means 228 of the indicator means 226 may be formed as a notch instead of a protrusion. The corresponding guidance means formed at the housing 12 may then be a corresponding axially extending rib.

In order to visualize the indicator means 226, the housing 12 of the present embodiment comprises a window 232. The window 232 is formed on a side surface of the housing 12 (Fig. 22).

The window 232 may be a cut out of the side wall. This cut out may be open or may be closed by a transparent pane for hygienic purposes.

In the present embodiment, two windows 232 are formed on diametrically oppositely sides, allowing for a good visibility of the protrusions 230 of the indicator means 226 from both sides of the dispenser 10. However, only one window 232 may be sufficient enough to see the indicator means 226. It is further noted that there may be more than two windows 232 formed on the housing 12, in particular its side wall, such as three, four, five or more windows. It is even further possible to configure the window such that it entirely extends in circumferential direction. Furthermore, the part of the housing 12 in which the indicator means 226 travels from fully retracted to fully extended position may be entirely transparent. In the context of the second embodiment of the dispenser 10 it is noted that the transmitter element 62 guided in the drive tube 136 may also form an indicator means 226 as described herein. The drive tube 136 and the part of the housing 12 in which the transmitter element 62 travels may then be transparent to make the indicator means 226 visible to the user.

In the present embodiment, the window 232 also forms a part of the guidance means. That is the window 232 comprises an axially extending groove. It is noted that the groove formed at the housing 12 and the groove formed at the window 232 transition into each other.

It is also noted that the window 232 may be also formed without any guidance means and the guidance means is then formed at an inner side of the housing 12 not comprising the window 232.

As can be seen from Fig. 23 in combination with Fig. 22, as the protrusions 230 also extend in the axial direction towards the rear end 70 of the indicator means 226, the protrusions 230 form a cover 234 hiding the spring 60 along the axial extension of the indicator means 226. That is, the spring 60 is received radially between the cover 234 and the main body 227 of the indicator means 226.

Furthermore, the protrusions 230 also form a guidance means for the spring, as parts of the spring are received radially between the outer wall of the main body 227 and the protrusions 230

In Fig. 22 a position of the indicator means 226 is shown in which the tank 16 is about half full. That is, the indicator means 226 is visible through the window 232, when the piston rod 50 is in a position between a fully retracted rear end position and a fully extended front end position.

The axial length of the window 232 can be dimensioned such that only the piston rod 50 is visible through the window 232, when the piston rod 50 is in its fully retracted rear end position (full tank 16). Furthermore, the axial length of the window 232 can be also dimensioned such that only the spring 60 is visible through the window 232, i.e. no indictor means 226 is visible, when the piston rod 50 is in its fully extended front end position (tank 16 entirely emptied or tank 16 detached from the dispenser 10).

In order to enhance the visibility, the piston rod 50 and/or the indicator means 226 may be colored. In particular, the piston rod 50 and the indicator means 226 may have the same color or the piston rod 50 and the indicator means 226 may be differently colored. A different coloration of the piston rod 50 and the indicator means 226 allows to better distinguish between the piston rod 50 and the indicator means 226 allowing for a better indication of the piston rod's axial position and therefore the filling status of the tank 16.

### List of reference signs

- 10: multi-use dispenser
- 11: front end
- 12: housing
- 12a: front housing part
- 12b: rear housing part
- 13: rear end
- 14: shielding
- 14a: front part of 14
- 14b: rear part of 14
- 16: tank
- 18: applicator tip
- 20: cannula
- 22: dispense activator
- 24: two-arm lever, lever
- 24a: fulcrum
- 27: stopping element
- 26: support structure
- 29: snap dome
- 28: push arm
- 30: push button of 24
- 32: drive arm of 24
- 34: drive nut
- 35: spring
- 36: rotation mechanism
- 38: ratchet teeth of 34
- 40: blocking element
- 42: ratchet teeth of 34
- 44: ratchet teeth of 40
- 46: spring
- 48: washer
- 50: threaded piston rod
- 52: dispensing mechanism
- 54: body nut
- 56: clutch
- 58: plunger
- 60: spring
- 62: transmitter element
- 64: inner end surface of 12
- 66: cavity
- 68: guidance structure
- 70: rear end
- 72: collar
- 71: groove
- 73: guidance means
- 74: engagement means of 54
- 76: engagement means of 56
- 78: teeth
- 80: teeth
- 82: passage
- 84: ramp feature
- 86: ramp of 84
- 88: ramp feature
- 90: ramp of 88
- 92: spring
- 94: shoulder
- 96: first coding means
- 98: second coding means
- 100: ring
- 102: groove
- 104: notch
- 106: protrusion
- 108: channel
- 110: protrusion
- 112: guide track
- 112a: first portion
- 112b: second portion
- 114: detent
- 116: stopping member
- 118: L-shaped groove
- 118a: axial portion of 118
- 118b: circumferential portion of 118
- 120: retaining means
- 120a: first retaining means
- 120b: second retaining means
- 122: pocket
- 124: interference means
- 126: spline
- 128: base
- 130: tine
- 132: break through
- 134: transmission means
- 136: drive tube
- 138: toothed engagement means
- 140: drive plate
- 142: shaft
- 144: bevel gear
- 146: bevel gear
- 148: passage
- 150: return spring
- 152: preload means
- 154: washer
- 156: collar
- 158: drive ratchet
- 160: splines
- 162: splines
- 164: shoulder
- 166: drive ratchet spring
- 168: ratchet teeth
- 170: ratchet teeth
- 172: ratchet teeth
- 174: ratchet teeth
- 176: counter ratchet
- 178: splines
- 180: splines
- 182: counter ratchet spring
- 184: collar
- 186: insert
- 188: splines
- 190: splines
- 192: clutch spring
- 194: reactor plate
- 196: neck
- 198: passage
- 200: front wall
- 202: side wall
- 204: cavity
- 206: connection member
- 208: corresponding connection member
- 210: plug
- 212: outlet opening
- 214: passage
- 216: predetermined breaking means
- 218: shell wall
- 220: radial gap
- 222: insert
- 224: connection means
- 226: indicator means
- 227: main body
- 228: guidance means
- 230: protrusion
- 232: window
- 234: cover
- B: arrow indicating pushing direction
- E: arrow indicating engagement direction
- I: arrow indicating an insertion direction
- R: arrow indicating rotation direction
- T: arrow indicting a twisting direction
- C1: arrow indicating a first deflection direction
- C2: arrow indicating a second deflection direction
- L: longitudinal axis
- P: pivot axis

## Claims

1. A dispenser (10) extending between a front end (11) and a rear end (13) along a longitudinal axis (L), the dispenser (10) comprising:
- a threaded piston rod (50) extending and movable along the longitudinal axis (L);
- a body nut (54) in engagement, in particular in threaded engagement, with the piston rod (50);
- a drive nut (34) in engagement, in particular in threaded engagement, with the piston rod (50); and
- a drive tube (136) rotatable about the longitudinal axis (L) and at least partially accommodating the piston rod (50) in a slidable non-engaged manner, the drive tube (136) interacting with the drive nut (34), wherein a rotation of the drive tube (136) in a first direction causes a rotation of the piston rod (50) about the longitudinal axis (L) by a rotation of the drive nut (34) and the rotation of the piston rod (50) being transferred into an axial movement of the piston rod (50), in particular towards the front end (11), by means of the engagement between the piston rod (50) and the body nut (54);
the dispenser (10) further comprising:
- a lever (24) pivotable about a pivot axis (P), wherein an activation of the lever (24) for dispense operation rotates the drive tube (136) in the first rotation direction.

2. The dispenser (10) of claim 1, wherein
the pivot axis (P) of the lever (24) and the longitudinal axis (L) are arranged at least substantially perpendicular to each other.

3. The dispenser (10) of claim 1 or 2, wherein
the dispenser (10) comprises transmission means (134) transferring motion between the lever (24) and the drive tube (136).

4. The dispenser (10) of claim 3, wherein
the transmission means (134) comprises a bevel gear (144) associated with the lever (24) and meshing with a corresponding bevel gear (146) associated with the drive tube (136), in particular formed on the drive tube (136).

5. The dispenser (10) of claim 4, wherein
two counter rotating bevel gears (144) are associated with the lever (24) and two counter rotating bevel gears (146) are associated with the drive tube (136), in particular on oppositely averted side surfaces of the drive tube (136).

6. The dispenser (10) of any one of the preceding claims, wherein
rotation of the drive tube (136) in the first direction is transmitted onto the drive nut (34) by means of a drive ratchet (158).

7. The dispenser (10) of claim 6, wherein
the drive ratchet (158) is rotationally fixed with regard to the drive tube (136) and the drive ratchet (158) comprising ratchet teeth (168) interacting with corresponding ratchet teeth (170) formed on the drive nut (34).

8. The dispenser (10) of any of the preceding claims, wherein
the drive tube (136) is rotated in the first rotation direction against a return force of a return spring (150), in particular the return spring (150) being a torsional spring; and/or wherein the lever (24) is activated against a return force of a return spring (150), in particular the return spring (150) being a compression spring.

9. The dispenser (10) of any of the preceding claims, wherein
the body nut (54) is rotationally fixed during dispense operation and/or the body nut (54) is allowed to freely rotate during resetting of the piston rod (50).

10. The dispenser (10) of any of the preceding claims, wherein
the dispenser (10) further comprises a clutch (56) moveable, in particular axially movable, between a disengaged position and an engaged position, the body nut (54) allowed to freely rotate in the disengaged position of the clutch (56) and the body nut (54) being rotationally fixed in the engaged position of the clutch.

11. The dispenser (10) of any of the preceding claims, wherein
the dispenser (10) comprises a counter ratchet (176) blocking a rotation of the drive nut (34) in a second direction opposite to the first direction of rotation.

12. The dispenser (10) of claim 11, wherein
the counter ratchet (176) rotationally fixing the drive nut (54) during resetting of the piston rod (50).

13. The dispenser (10) of any one of claims 11 and 12, wherein
the respective blocking directions of the drive ratchet (158) and the counter ratchet (176) are effective in the same direction.

14. The dispenser (10) of any of the preceding claims, wherein
a spring (60) biases the piston rod (50) towards the front end (11), in particular the spring (60) being at least partially, preferably entirely, accommodated in the drive tube (136).

15. The dispenser (10) of any of the preceding claims, wherein
the piston rod (50) is in threaded engagement with each of the drive nut (34) and the body nut (54), the respective threads of the drive nut (34) and the body nut (54) being counter directed and a pitch of the drive nut thread is the same as or different to a pitch of the body nut thread, in particular a drive nut thread being larger than a body nut thread.
